# EUROPEAN PATENT APPLICATION

(11) **EP 4 501 299 A1**
(43) Date of publication of application: **05.02.2025**
(21) Application number: 23811395.5
(22) Date of filing: 07.03.2023
(51) Int. Cl.: A61H 33/00, A47K 3/00, F24H 15/176, A61B 5/0245, A61B 5/26, A61B 5/28

(54) **BATHTUB DEVICE**

(30) Priority: 25.05.2022 JP 2022085000; 10.01.2023 JP 2023001452
(71) Applicant: TOTO LTD., Kokurakita-ku Kitakyushu-shi, Fukuoka 802-8601 (JP)
(72) Inventor: TSUBOI Hiroshi, Kitakyushu-shi, Fukuoka 802-8601 (JP); ICHIKAWA Yu, Kitakyushu-shi, Fukuoka 802-8601 (JP); ENOKI Masayoshi, Kitakyushu-shi, Fukuoka 802-8601 (JP); WATANABE Kei, Kitakyushu-shi, Fukuoka 802-8601 (JP); JINDE Mao, Kitakyushu-shi, Fukuoka 802-8601 (JP); UNO Ryoko, Kitakyushu-shi, Fukuoka 802-8601 (JP); TAIRA Rina, Kitakyushu-shi, Fukuoka 802-8601 (JP); ISHII Yuwa, Kitakyushu-shi, Fukuoka 802-8601 (JP)
(74) Representative: Bandpay & Greuter
(86) International application number: PCT/JP2023/008611
(87) International publication number: WO 2023/228514

(57) **Abstract**

There is provided a bathtub apparatus in which a bather can confirm objective information in association with subjective information by history information based on a plurality of bathing histories. A bathing information system of the present invention includes a bather specifying unit 42, an information acquisition unit 44 that acquires objective information, and program 50 that controls a controller to store subjective information input by the bather in the controller, the program causing a controller 52 to store a bathing history obtained by associating objective information A acquired by the information acquisition unit regarding specific bathing of a specified bather with subjective information B input by the bather specified for the specific bathing and causing the controller 52 to implement an output function of outputting, to an output device, history information C based on a plurality of bathing histories for the specified bather.

## Description

### BACKGROUND OF THE INVENTION

### Field of the Invention

The present invention relates to a bathing information system, particularly to a bathing information system for acquiring information on a bather bathing in a bathtub.

### Description of the Related Art

Conventionally, as described in Japanese Patent Laid-Open No. 2005-334394, for example, a bathing management system that attempts to effectively manage bathing by informing a bather soaked in a bathtub of electrocardiogram information of the bather is known. In this bathing management system, a heart rate or a heart rate rise of the bather is displayed.

Furthermore, for example, Japanese Patent Laid-Open No. 2019-200448 discloses that a bathing navigation system includes a wearable device that a user wears on an arm or the like while bathing. This wearable device presents information on risk and comfort of bathing for the user based on feature data, including biological information, medical knowledge about bathing, and subjective knowledge as in SNS articles by a bathing experiencer and others, and encourages guidance of the user to a more appropriate bathing environment and bathing facility.

Additionally, for example, Japanese Patent Laid-Open No. 2020-106964 discloses that when a bathing system determines that sleep after bathing is good with reference to sleep information input by the bather, a perceived rising target temperature is set in the same manner as at a previous bathing time.
Patent Literature 1: Japanese Patent Laid-Open No. 2005-334394
Patent Literature 2 : Japanese Patent Laid-Open No. 2019-200448
Patent Literature 3: Japanese Patent Laid-Open No. 2020-106964

In the invention described in Patent Literature 1, a bather is informed only of obj ective information such as a heart rate or a heart rate rise of the bather, and hence a relationship between the objective information and bather's subjectivity is difficult for the bather to understand. A bathing style and bather's subjectivity vary with the bather, and hence there is a problem that even if the objective information indicates a satisfactory condition, the bather may feel discomfort.

Furthermore, in the invention described in Patent Literature 2, although information such as risk of bathing is provided based on subjective knowledge as in SNS articles by a bathing experiencer and others in addition to biological information from a wearable device and medical knowledge regarding bathing, information such as general risk is only provided. Therefore, in the invention of Patent Literature 2, there are problems that a specified bather cannot know what subjective information was in a plurality of bathing histories of the bather, and cannot know what subj ective information was for a specific bathing purpose in the plurality of bathing histories and that it is difficult for the specified bather to achieve more comfortable bathing of the specified bather by comparing the subjective information of the specified bather with objective information and according to judgement of the specified bather.

Additionally, in the invention described in Patent Literature 3, there is a problem that when it is determined that sleep after bathing is good, a bathing system only sets a perceived rising target temperature in the same manner as in the previous bathing time and hence cannot help a bather to know a bathing method suitable for the bather by comparing bather's subjective information with the obj ective information. Furthermore, in the invention of Patent Literature 3, the bathing system sets the perceived rising target temperature based on its flow, and hence there is a problem that it is difficult for the bather to try more comfortable bathing according to history information obtained by associating objective information with subjective information.

Therefore, an object of the present invention is that a bather can confirm objective information in association with subjective information by history information based on a plurality of bathing histories and that the bather can easily achieve more comfortable bathing.

### SUMMARY OF THE INVENTION

In order to solve the above-described problems, the present invention provides a bathing information system for acquiring information on a bather bathing in a bathtub, the bathing information system including a bather specifying unit that specifies who the bather is for specific bathing, an information acquisition unit that acquires objective information on the bathing of the bather, and program that controls a controller to store subjective information input by the bather in the controller, the program causing the controller to store a bathing history obtained by associating the objective information acquired by the information acquisition unit regarding the specific bathing of the specified bather with the subjective information input by the bather specified for the specific bathing and causing the controller to implement an output function of outputting, to an output device, history information based on a plurality of bathing histories for the specified bather.

According to the present invention including such a configuration as described above, the program causes the controller to store the bathing history obtained by associating the objective information acquired by the information acquisition unit regarding the specific bathing of the specified bather with the subjective information input by the bather specified for the specific bathing and to implement an output function of outputting, to the output device, the history information based on the plurality of bathing histories for the specified bather. Consequently, the bather can confirm the objective information in association with the subjective information by the history information based on the plurality of bathing histories, and the system can help the bather to know a bathing method suitable for the bather by comparing the subjective information with the objective information in the plurality of bathing histories, which can make it easier for the bather to achieve more comfortable bathing.

In one embodiment of the present invention, preferably, the objective information includes a temperature of warm water in the bathtub and a bathing time.

According to the embodiment of the present invention including such a configuration as described above, the objective information includes the warm water temperature and bathing time indicating a bathing environment, and the bather can confirm the warm water temperature and bathing time in association with the subjective information by the history information based on the plurality of bathing histories. The present system can help the bather to know the bathing method suitable for the bather by comparing the subjective information of the bather with the warm water temperature and bathing time in the plurality of bathing histories and can make it easier for the bather to achieve more comfortable bathing.

In one embodiment of the present invention, preferably, the objective information includes biological information of the bather.

According to the embodiment of the present invention including such a configuration as described above, the objective information includes the biological information of the bather, and the bather can confirm the biological information of the bather that is a reaction of bather's body in association with the subjective information by the history information based on the plurality of bathing histories. The present system can help the bather to know the bathing method suitable for the bather by comparing the bather's subjective information with the biological information of the bather that is the reaction of the bather's body in the plurality of bathing histories and can make it easier for the bather to achieve more comfortable bathing.

In one embodiment of the present invention, preferably, the program further causes the controller to implement a function of outputting, to the output device, preset recommended information together with the history information.

According to the embodiment of the present invention including such a configuration as described above, the controller performs the recommended information output function of outputting, to the output device, the preset recommended information together with the history information. Consequently, the bather can compare the preset recommended information with the history information of the bather, can be helped in adjusting the bathing method of the bather, and can try the bathing method within the range of the preset recommended information.

In one embodiment of the present invention, preferably, the history information includes recommended range information determined based on the plurality of bathing histories.

According to the embodiment of the present invention including such a configuration as described above, the history information includes the recommended range information determined based on the plurality of bathing histories. Consequently, the present system can help the bather to know the bathing method more suitable for the bather while comparing the subjective information of the bather with the objective information by the recommended range information and can further make it easier for the bather to achieve more comfortable bathing.

In one embodiment of the present invention, preferably, the output function includes a first output function and a second output function, and the program causes the controller to store a first bathing history obtained by associating first objective information selected for a first analysis application from the objective information with first subjective information input for the first analysis application from the subjective information and causes the controller to implement the first output function of outputting, to the output device, first history information based on a plurality of first bathing histories for a specified bather. The program causes the controller to store a second bathing history obtained by associating second objective information selected for a second analysis application from the objective information with second subjective information input for the second analysis application from the subjective information and causes the controller to implement the second output function of outputting, to the output device, second history information based on a plurality of second bathing histories for the specified bather. The program further causes the controller to implement a function of switching between the first output function and the second output function.

According to the embodiment of the present invention including such a configuration as described above, the controller can output the first history information for the first analysis application to the output device by the first output function, can output the second history information for the second analysis application to the output device by the second output function and can further switch between the first output function and the second output function by the switching function. Consequently, the present system can help the bather to know the bathing method suitable for the bather while switching and comparing history information for different analysis applications by the switching function and can make it easier for the bather to achieve more comfortable bathing.

According to one embodiment of the present invention, a bathing information system for acquiring information on a bather bathing in a bathtub, the system including a bather specifying unit that specifies who the bather is for specific bathing, an information acquisition unit that acquires objective information on bathing of the bather, an input unit that accepts input of subjective information, a controller that stores the objective information and the subjective information; and a display that displays information received from the controller, and the controller may store a bathing history obtained by associating the objective information acquired for the specific bathing of the specified bather by the information acquisition unit with the subjective information input in the input unit regarding the bather specified for the specific bathing, and may display, on the display, history information based on a plurality of bathing histories for the specified bather.

According to the embodiment of the present invention including such a configuration as described above, the controller stores the history information obtained by associating the objective information acquired by the information acquisition unit for the specific bathing of the specified bather with the subjective information input in the input unit regarding the bather specified for the specific bathing, and displays, on the display, the history information based on the plurality of bathing histories for the specified bather. Consequently, the bather can confirm the objective information in association with the subjective information by the history information based on the plurality of bathing histories. The present system can help the bather to know the bathing method suitable for the bather by comparing subjective information with the objective information in the plurality of bathing histories and can make it easier for the bather to achieve more comfortable bathing.

In the present invention, preferably, the program presumes subjective information corresponding to a value of objective information to which any subjective information is not tied, based on objective information on at least one bathing, and subjective information tied to the value of the objective information and causes the output device to output presumed subjective information.

According to the present invention including such a configuration as described above, since the program presumes the subjective information corresponding to the value of the objective information to which any subjective information is not tied, it is possible to presume subjective information indicating, for example, whether the bather feels warmed sufficiently or whether the bather perspires, from values of objective information including bathing conditions that are not experienced by the bather and biological information of the bather. As a result, it is possible to recommend the value of objective information that may satisfy the bather even for the value of objective information in a region that is not experienced by the bather and to provide comfortable bathing.

In the present invention, preferably, examples of the value of the obj ective information include a temperature of warm water in the bathtub, and a bathing time.

According to the present invention including such a configuration as described above, it is possible to presume how the bather feels (subjective information) for the temperature of warm water and the bathing time, for which the bather has not input any subjective information, based on the temperature of warm water and the bathing time in the bathtub, which are objective information, and based on the subjective information corresponding to the objective information. Consequently, for example, even if the bather is bathing in warm water at a temperature for which the bather has not input any subjective information, it is possible to presume how much bathing time is taken to obtain sufficient feeling of warmth and to recommend bathing conditions that can satisfy the bather.

In the present invention, preferably, the examples of the value of the objective information further include a temperature in a room in which the bathtub is provided.

According to the present invention including such a configuration as described above, the examples of the value of the objective information include the temperature in the room in which the bathtub is provided, and it is therefore possible to precisely recommend the bathing conditions that can satisfy the bather, even if subjectivity (satisfaction level) of the bather changes depending on season, weather on the day of bathing, or the like.

In the present invention, preferably, the examples of the value of the objective information further include a water level of warm water in the bathtub.

According to the present invention including such a configuration as described above, since the examples of the value of the objective information include the water level of warm water in the bathtub, it is possible to add a difference in feeling of warmth felt by the bather between half-body bathing and full-body bathing to presumption of subjective information and to precisely recommend the bathing conditions that can satisfy the bather.

According to the bathing information system of the present invention, the bather can confirm the objective information in association with the subjective information by the history information based on the plurality of bathing histories, and the system can make it easier for the bather to achieve more comfortable bathing.

### BRIEF DESCRIPTION OF THE DRAWINGS

FIG. 1 is a perspective view showing an entire bath room in which a bathing information system according to a first embodiment of the present invention is installed;
FIG. 2 is a perspective view of a bathtub apparatus of the bathing information system according to the first embodiment of the present invention viewed diagonally from above;
FIG. 3 is a view illustrating a warm water supply system to the bathtub apparatus of the bathing information system according to the first embodiment of the present invention;
FIG. 4 is a diagram illustrating a schematic configuration of a signal processing device and a remote controller in the bathing information system according to the first embodiment of the present invention;
FIG. 5 is a view schematically illustrating a positional relationship between bather's body leaning on a backrest surface of a bathtub body of the bathing information system according to the first embodiment of the present invention and each electrode attached to the backrest surface;
FIG. 6 is a diagram showing that history information based on a bathing history obtained by associating objective information with subjective information is displayed on terminal equipment in the bathing information system according to the first embodiment of the present invention;
FIG. 7 is a diagram showing that the history information based on the bathing history obtained by associating the objective information with the subjective information is displayed on the terminal equipment in the bathing information system according to the first embodiment of the present invention;
FIG. 8 is a diagram showing that the history information based on the bathing history obtained by associating the objective information with the subjective information is displayed on the terminal equipment in the bathing information system according to the first embodiment of the present invention;
FIG. 9 is a diagram showing that the history information based on the bathing history obtained by associating the objective information with the subjective information is displayed on the terminal equipment in the bathing information system according to the first embodiment of the present invention;
FIG. 10 is a diagram showing that the history information based on the bathing history obtained by associating the objective information with the subjective information is displayed on the terminal equipment in the bathing information system according to the first embodiment of the present invention;
FIG. 11 is a diagram showing that the history information based on the bathing history obtained by associating the objective information with the subjective information is displayed on the terminal equipment in the bathing information system according to the first embodiment of the present invention;
FIG. 12A is a diagram showing that the history information based on the bathing history obtained by associating the objective information with the subjective information is displayed in a form of graph on the terminal equipment, in the bathing information system according to the first embodiment of the present invention;
FIG. 12B is a diagram showing that the history information based on the bathing history obtained by associating the objective information with the subjective information is displayed in a form of table on the terminal equipment, in the bathing information system according to the first embodiment of the present invention;
FIG. 12C is a diagram showing that the history information based on the bathing history obtained by associating the objective information with the subjective information is displayed in a form of graph and a form of text on the terminal equipment, in the bathing information system according to the first embodiment of the present invention;
FIG. 13A is a diagram showing that the history information based on the bathing history obtained by associating the objective information with the subjective information is displayed in a form of graph on the terminal equipment, in the bathing information system according to the first embodiment of the present invention;
FIG. 13B is a diagram showing that the history information based on the bathing history obtained by associating the objective information with the subjective information is displayed in a form of table on the terminal equipment, in the bathing information system according to the first embodiment of the present invention;
FIG. 13C is a diagram showing that the history information based on the bathing history obtained by associating the objective information with the subjective information is displayed in a form of graph and a form of text on the terminal equipment, in the bathing information system according to the first embodiment of the present invention;
FIG. 14A is a diagram showing that the history information based on the bathing history obtained by associating the objective information with the subjective information is displayed in a form of graph on the terminal equipment, in the bathing information system according to the first embodiment of the present invention;
FIG. 14B is a diagram showing that the history information based on the bathing history obtained by associating the objective information with the subjective information is displayed in a form of table on the terminal equipment, in the bathing information system according to the first embodiment of the present invention;
FIG. 14C is a diagram showing that the history information based on the bathing history obtained by associating the objective information with the subjective information is displayed in a form of graph and a form of text on the terminal equipment, in the bathing information system according to the first embodiment of the present invention;
FIG. 15 is a perspective view showing an entire bath room in which a bathing information system according to a second embodiment of the present invention is installed;
FIG. 16 is a diagram illustrating a schematic configuration of a signal processing device and a remote controller of the bathing information system according to the second embodiment of the present invention;
FIG. 17 is a diagram illustrating an example of bathing history acquired in a bathing information system of a third embodiment of the present invention;
FIG. 18 is a graph illustrating an example of a bathing history representing subjective information corresponding to a temperature of warm water and a bathing time, in the bathing information system of the third embodiment of the present invention;
FIG. 19 is a diagram showing an example of a heat map representing subjective information corresponding to a temperature of warm water and a bathing time presumed based on the bathing history, in the bathing information system of the third embodiment of the present invention;
FIG. 20 is a diagram showing an example of a procedure of presuming subjective information corresponding to a value of objective information to which any subjective information is not tied, based on the acquired bathing history, in the bathing information system of the third embodiment of the present invention;
FIG. 21 is a diagram showing an example of the procedure of presuming the subjective information corresponding to the value of the objective information to which any subjective information is not tied, based on the acquired bathing history, in the bathing information system of the third embodiment of the present invention;
FIG. 22 is a diagram showing an example of the procedure of presuming the subjective information corresponding to the value of the objective information to which any subjective information is not tied, based on the acquired bathing history, in the bathing information system of the third embodiment of the present invention;
FIG. 23 is a view illustrating an example of a screen displayed on terminal equipment before a bather starts bathing, in the bathing information system of the third embodiment of the present invention;
FIG. 24 is a view illustrating an example of a screen displayed on a remote controller while the bather is bathing, in the bathing information system of the third embodiment of the present invention;
FIG. 25 is a view schematically illustrating an operation of a bathing information system according to a fourth embodiment of the present invention;
FIG. 26 is a view illustrating an example of a screen in which a bather inputs subj ective information, in a bathing information system according to a fifth embodiment of the present invention;
FIG. 27 is a diagram illustrating an example of a screen displayed on bather's smartphone before bathing, in the bathing information system according to the fifth embodiment of the present invention;
FIG. 28 is a view illustrating an example of a screen displayed on a remote controller during bathing, in the bathing information system according to the fifth embodiment of the present invention;
FIG. 29 is a view illustrating an example of a screen in which a bather inputs subj ective information, in a bathing information system according to a sixth embodiment of the present invention;
FIG. 30 is a view illustrating an example of a gauge displayed on a remote controller screen in a bath room, in the bathing information system according to the sixth embodiment of the present invention;
FIG. 31 is a view illustrating an example of a screen in which a bather inputs subj ective information, in a bathing information system according to a seventh embodiment of the present invention;
FIG. 32 is a view illustrating an example of a gauge displayed on a remote controller screen in a bath room, in the bathing information system according to the seventh embodiment of the present invention;
FIG. 33 is a diagram illustrating an example of an image captured by a thermo-sensor for acquiring a body temperature of a bather, in a bathing information system according to an eighth embodiment of the present invention;
FIG. 34 is a view illustrating an example of a screen in which the bather inputs subjective information, in the bathing information system according to the eighth embodiment of the present invention;
FIG. 35 is a view illustrating an example of a gauge displayed on a remote controller screen in a bath room, in the bathing information system according to the eighth embodiment of the present invention; and
FIG. 36 is a view illustrating an example of a gauge displayed on a remote controller screen in a bath room, in a bathing information system according to a ninth embodiment of the present invention.

### DETAILED DESCRIPTION OF THE INVENTION

Next, a bathing information system according to a first embodiment of the present invention will be described with reference to the accompanying drawings.

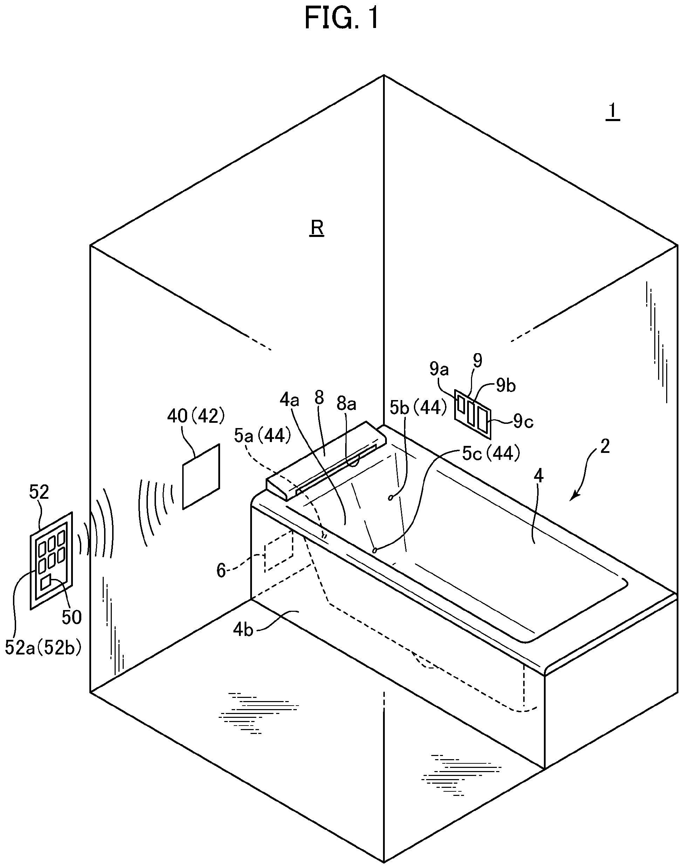

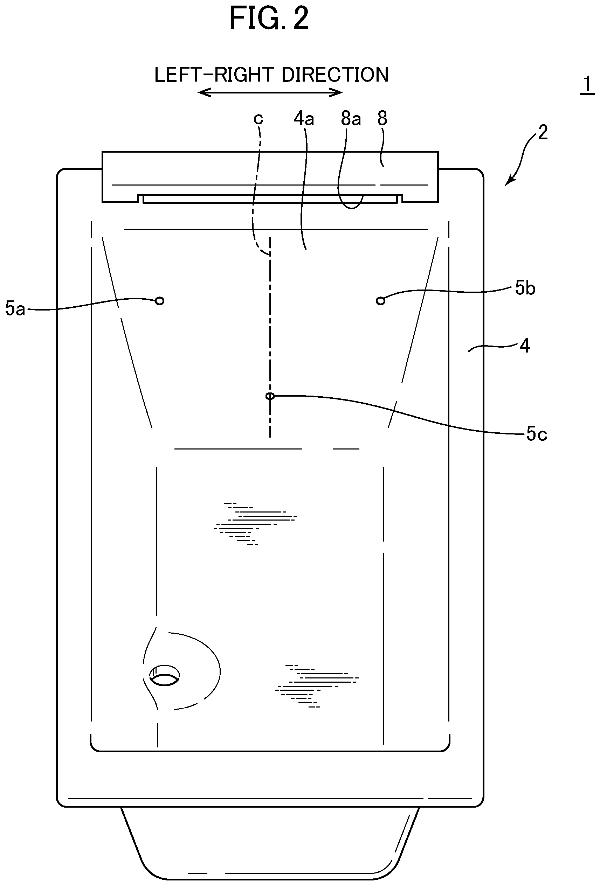

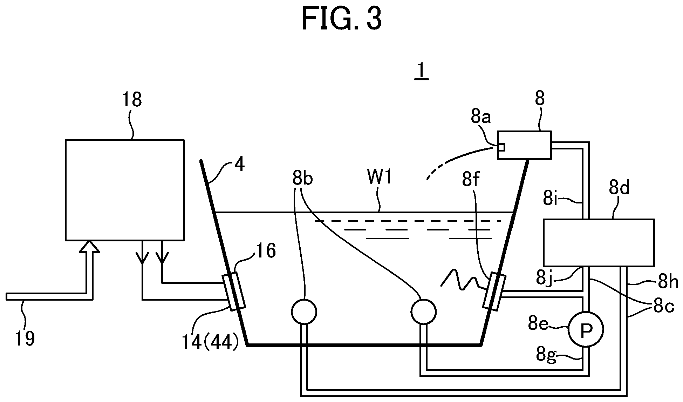

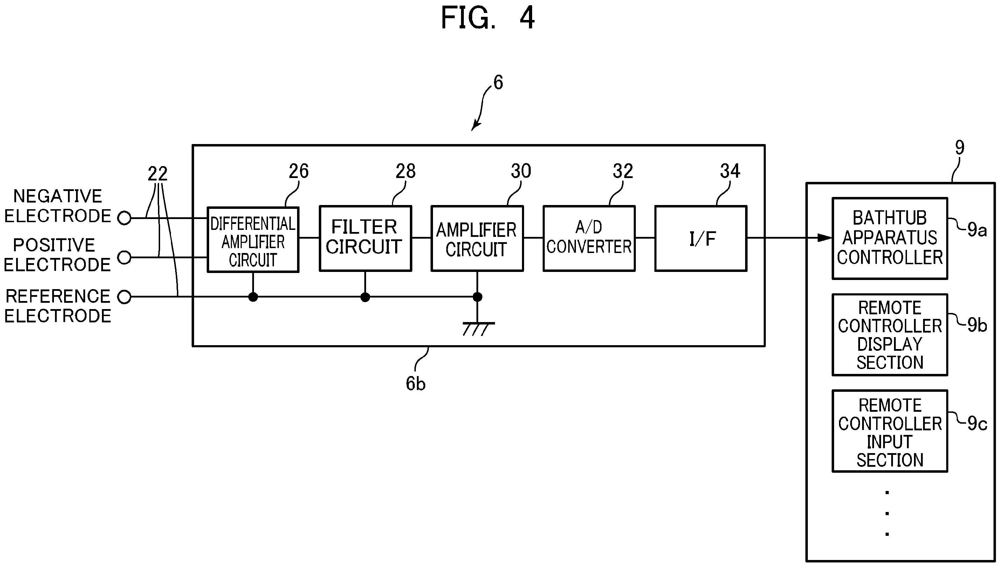

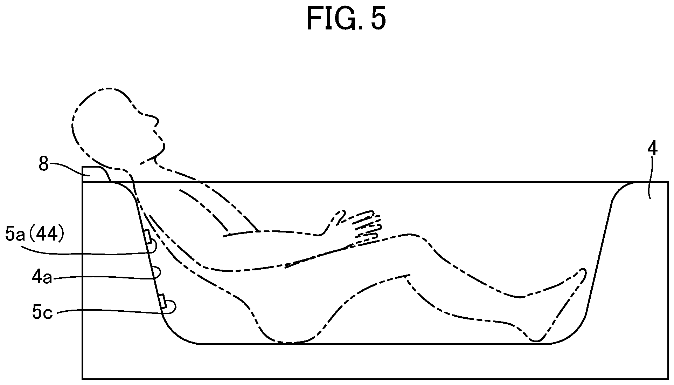

FIG. 1 is a perspective view showing an entire bath room in which the bathing information system according to the first embodiment of the present invention is installed, FIG. 2 is a perspective view of a bathtub apparatus of the bathing information system according to the first embodiment of the present invention viewed diagonally from above, FIG. 3 is a view showing a warm water supply system to the bathtub apparatus of the bathing information system according to the first embodiment of the present invention, FIG. 4 is a diagram showing a schematic configuration of a signal processing device and a remote controller in the bathing information system according to the first embodiment of the present invention, and FIG. 5 is a view schematically showing a positional relationship between bather's body leaning on a backrest surface of a bathtub body of the bathing information system according to the first embodiment of the present invention and each electrode attached to the backrest surface.

As shown in FIG. 1, a bathing information system 1 of the first embodiment of the present invention acquires information on a bather bathing in a bathtub. The bathing information system 1 includes a bathtub apparatus 2 installed in a bath room R. The bathtub apparatus 2 includes a bathtub body 4, a negative electrode 5a, a positive electrode 5b and a reference electrode 5c that are attached to the bathtub body 4, a signal processing device 6 that processes signals acquired by the electrodes, a water discharge device 8 provided on a top edge of the bathtub body 4, and a remote controller 9 attached to a wall surface of the bath room R. In the present embodiment, the bathtub apparatus 2 is disposed in contact with the wall surface on one side of the bath room R.

The bathtub body 4 is formed in a substantially rectangular box shape in planar view and is configured to store warm water in an interior. In the present embodiment, the bathtub body 4 is disposed so that one long side and two short sides on opposite sides of the long side are entirely in contact with an inner wall surface of the bath room R. Furthermore, an inner wall surface constituting one short side of the bathtub body 4 is configured as a backrest surface 4a that is a first inner wall surface formed for the bather to lean on. Additionally, an apron 4b is detachably attached to the long side of the bathtub body 4 on a side that is not in contact with the inner wall surface of the bath room R. Here, the backrest surface is an inner wall surface that the back of the bather touches during bathing. To the bathtub body 4, warm water at a predetermined temperature is supplied from a water heater 18 (see FIG. 3). The water heater 18 is connected to a water supply 19 as an external supply source.

The water discharge device 8 is provided in an upper part of the backrest surface 4a of the bathtub body 4 and is configured to discharge warm water toward the inside of the bathtub body 4. As shown in FIG. 2, in the present embodiment, the water discharge device 8 includes a flat and wide water discharge port 8a extending along the top edge of the backrest surface 4a and is configured to discharge warm water toward shoulders of the bather leaning on the backrest surface 4a.

Furthermore, as shown in FIG. 3, the water discharge device 8 includes a pump 8e that sucks in warm water stored in the bathtub body 4 from water intake parts 8b and discharges warm water from the water discharge port 8a via a circulation flow channel 8c and a chamber 8d. Consequently, warm water in the bathtub body 4 is sucked in by the pump 8e, discharged from the water discharge port 8a, and circulated to flow into the bathtub body 4. The circulation flow channel 8c is branched out to a flow channel on a side of a bathtub water discharge port 8f provided on the backrest surface 4a of the bathtub body 4 on a downstream side of the pump 8e and to a flow channel extending on a chamber 8d side. Therefore, part of warm water sucked in from the water intake parts 8b is discharged from the bathtub water discharge port 8f of the bathtub body 4 via the circulation flow channel 8c.

The circulation flow channel 8c further includes an injection part side flow channel 8g provided with the pump 8e, an introduction flow channel 8h connected to the chamber 8d and introducing warm water inside the bathtub body 4 into the chamber 8d, and a water discharge device side flow channel 8i extending from the chamber 8d to a water discharge device 8 side.

At an outlet of the injection part side flow channel 8g to the chamber 8d, a jet nozzle 8j is provided. The jet nozzle 8j forms an injection part that is disposed downstream of the pump 8e and that injects warm water supplied from the pump 8e to pass the warm water through the chamber 8d. The warm water injected by the jet nozzle 8j draws warm water inside the chamber 8d and warm water from the introduction flow channel 8h by a jet pump operation, and a flow rate of warm water drawn from the chamber 8d and the introduction flow channel 8h is added to a flow rate of warm water injected from the jet nozzle 8j. Thus, a larger flow rate of warm water is supplied to the water discharge device side flow channel 8i. The water discharge device side flow channel 8i extends onto an extension line of the jet nozzle 8j. In the chamber 8d, when warm water injected by the jet nozzle 8j passes through the chamber 8d, warm water in the chamber 8d is drawn into the injected warm water to form a larger flow rate of stream.

Furthermore, in the present embodiment, since the water discharge port 8a of the water discharge device 8 is formed flat and wide, warm water discharged from the water discharge port 8a is discharged as a wide strip of water film, and lands on a position away from the backrest surface 4a in a water surface of warm water stored in the bathtub body 4.

As shown in FIGS. 2 and 5, the negative electrode 5a and the positive electrode 5b are a set of electrodes attached to the bathtub body 4 for detecting an electrocardiogram signal of the bather via warm water stored in the bathtub body 4. The negative electrode 5a and the positive electrode 5b function as an information acquisition unit 44 described later. The electrocardiogram signal of the bather is acquired as a signal of a potential difference between the negative electrode 5a and the positive electrode 5b. A set of electrodes including the negative electrode 5a and the positive electrode 5b are all arranged on the backrest surface 4a constituting the short side of the bathtub body 4. Furthermore, the reference electrode 5c is an electrode for acquiring a reference potential for potentials detected by the negative electrode 5a and the positive electrode 5b, and this electrode is also disposed on the backrest surface 4a of the bathtub body 4. The reference electrode 5c may also function as the information acquisition unit 44 described later.

Thus, in the bathtub apparatus 2 of the present embodiment, all the electrodes for acquiring the electrocardiogram signal are arranged on one backrest surface 4a. In other words, in the bathtub apparatus 2 of the present embodiment, the electrocardiogram signal is detected only by the electrodes arranged on the one backrest surface 4a.

As shown in FIG. 2, the negative electrode 5a and the positive electrode 5b are arranged at left and right symmetric positions with respect to a center line c, on left and right of the center line c of the backrest surface 4a in a horizontal direction (width direction). That is, the negative electrode 5a and the positive electrode 5b are each arranged away from the center line c in a left-right direction on the backrest surface 4a, and the negative electrode 5a and the positive electrode 5b are attached to height positions below the water surface in the bathtub body 4 and above the reference electrode 5c.

In contrast, the reference electrode 5c is disposed on the center line c of the backrest surface 4a below the negative electrode 5a and the positive electrode 5b. The reference electrode 5c does not necessarily have to be provided and may be omitted. The electrocardiogram signal acquired by the negative electrode 5a and the positive electrode 5b is transmitted to the signal processing device 6 through conductor wires 22 (see FIG. 4) extending from each of the negative electrode 5a and the positive electrode 5b, and the electrocardiogram signal is input to the signal processing device 6. Furthermore, the reference electrode 5c is also connected to the signal processing device 6 via a conductor wire 22. The conductor wires 22 extending from the respective electrodes and the signal processing device 6 to which these conductor wires 22 are connected are all accommodated in a space behind the backrest surface 4a of the bathtub body 4. Alternatively, the conductor wires 22 extending from the respective electrodes and the signal processing device 6 to which these conductor wires 22 are connected may be accommodated in an external space of the bath room such as a ceiling back of the bath room R.

Next, a configuration of the signal processing device 6 and the remote controller 9 will be described with reference to FIG. 4.

The signal processing device 6 is an electrical circuit to which the negative electrode 5a, the positive electrode 5b, and the reference electrode 5c are connected, and is disposed on a back side of the backrest surface 4a of the bathtub body 4. The signals acquired by the negative electrode 5a, the positive electrode 5b, and the reference electrode 5c are processed by the signal processing device 6, and the processing result is displayed on the remote controller 9 disposed in the bath room R.

The signal processing device 6 includes a differential amplifier circuit 26, a filter circuit 28, an amplifier circuit 30, an A/D converter 32, and an interface circuit 34, and these circuits are built in a housing 6b. The differential amplifier circuit 26 is an amplifier circuit to which the conductor wires 22 extending from each of the negative electrode 5a and the positive electrode 5b are connected, and is configured to amplify a differential voltage between the negative electrode 5a and the positive electrode 5b.

The filter circuit 28 is configured such that the differential voltage amplified by the differential amplifier circuit 26 is input, an unnecessary frequency band component such as hum is eliminated, and a signal component in a required frequency band is passed.

The amplifier circuit 30 is configured such that the signal from which the unnecessary frequency band component such as hum is eliminated by the filter circuit 28 is input and the input signal is amplified. Furthermore, the conductor wire 22 extending from the reference electrode 5c is connected to the ground of the differential amplifier circuit 26, the filter circuit 28, and the amplifier circuit 30.

The A/D converter 32 is configured to convert an analog signal amplified by the amplifier circuit 30 into a digital signal.

The interface circuit 34 is configured such that a digital signal A/D converted by the A/D converter 32 is input and the input digital signal is sent to the remote controller 9 and/or an external display device (not shown).

The remote controller 9 has a control function capable of controlling supply of warm water from the water heater 18 to the bathtub apparatus 2 and can operate an electrocardiogram signal detecting function of the bathtub apparatus 2 and the water discharge device 8. The remote controller 9 includes a bathtub apparatus controller 9a capable of controlling the supply of warm water to the bathtub apparatus 2, a remote controller display section 9b that displays a bathing history, history information, and the like, as an output device, and a remote controller input section 9c that accepts input of a command to the bathtub apparatus controller 9a.

The bathtub apparatus controller 9a includes a built-in CPU, memory, and the like and controls equipment connected to control the supply of warm water from the water heater 18 to the bathtub apparatus 2, and the bathtub apparatus 2 based on predetermined control program recorded in the memory or the like. The bathtub apparatus controller 9a is electrically connected to the water heater 18, the signal processing device 6, the pump 8e of the water discharge device 8, a temperature sensor 14, the remote controller display section 9b, the remote controller input section 9c, and the like. These electrical connections may be performed by wireless communication or the like. Furthermore, the bathtub apparatus controller 9a is connected to terminal equipment 52 such as bather's smartphone by wireless communication (for example, communication via internet) or the like. The bathtub apparatus controller 9a bidirectionally communicates with the terminal equipment 52 and receives, for example, a command necessary for displaying on the remote controller display section 9b from the terminal equipment 52.

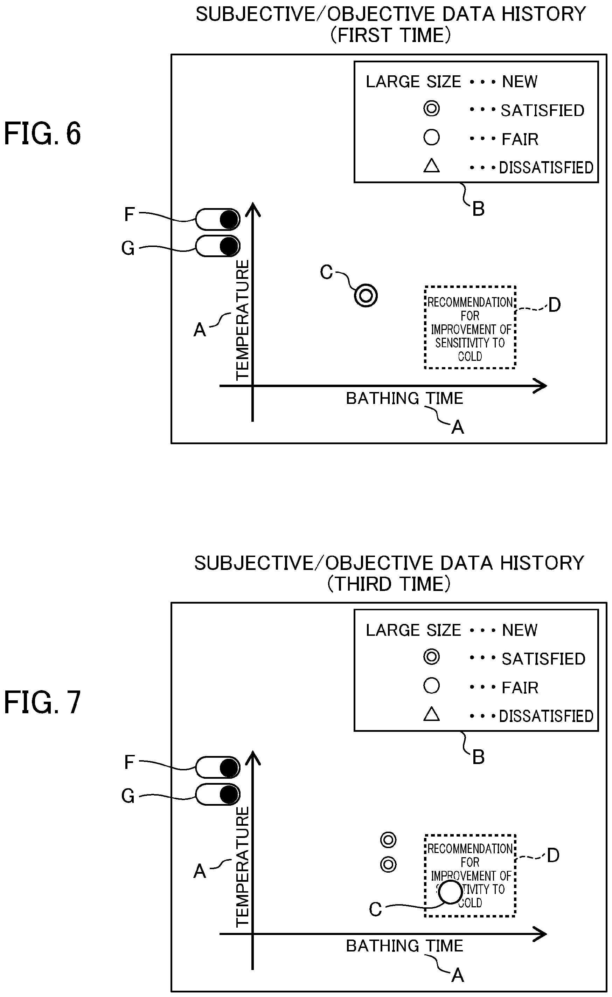

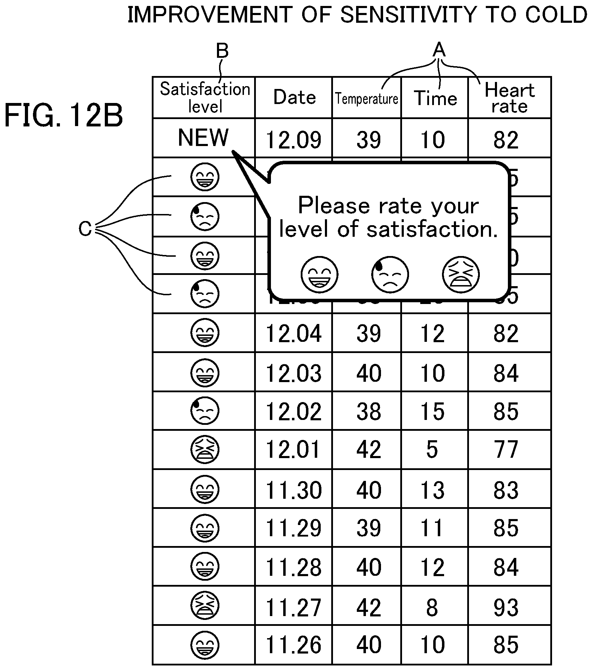

The bathtub apparatus controller 9a acquires and records signals sent via the signal processing device 6 from the negative electrode 5a, the positive electrode 5b and the like. Therefore, the negative electrode 5a, the positive electrode 5b and the like acquire a heart rate and bathing time in objective information A on the bathing of the bather (see FIGS. 6, 12B, etc.). The bathtub apparatus controller 9a calculates the heart rate of the bather based on the signal sent from the signal processing device 6, as a heart rate conversion unit. The bathtub apparatus controller 9a can recognize start of bathing by measurement start of the bather's heart rate and can recognize end of the bathing by end of the heart rate measurement. Therefore, the bathing time is a time from when the bather starts entering the bathtub to when the bather finishes bathing by exiting the bathtub. In addition, the bather may enter the bathtub once (first bathing), exit the bathtub to wash the body, and enter the bathtub again (second bathing). For example, when a period between the first bathing and the second bathing is less than a predetermined time of, for example, 15 minutes, the bathing time is a time obtained by adding up a first bathing time and a second bathing time. For example, when the period between the first bathing and the second bathing is a predetermined time of, for example, 15 minutes or more, the bathing time is a longer time of the first bathing time and the second bathing time.

Furthermore, the bathtub apparatus controller 9a acquires and records a temperature of warm water in the bathtub body 4 from the temperature sensor 14. The bathtub apparatus controller 9a transmits the acquired objective information A to the terminal equipment 52. Therefore, information based on the obj ective information A can be displayed also on a terminal equipment 52 side described later. Additionally, the present invention may be configured such that part of the function of the bathtub apparatus controller 9a is incorporated into the terminal equipment 52 and a display device (not shown) disposed outside the bath room R.

The remote controller display section 9b forms a display section such as a monitor disposed in the bath room R. The remote controller display section 9b may include the display device disposed outside the bath room R.

The remote controller input section 9c is configured to accept input of a command to control the bathtub apparatus controller 9a. The input to the remote controller input section 9c may implement an output function of program 50 to output history information onto the remote controller display section 9b of the remote controller 9 that is an output device. Such an output function is not limited to the remote controller display section 9b and may be implemented by outputting information as audio through a speaker or the like that generates audio output.

The bathtub apparatus 2 further includes a bather specifying unit 42 (pairing device 40) that specifies who the bather is for specific bathing by comparison with registered information or the like, and the information acquisition unit 44 (temperature sensor 14 or the like) that acquires objective information on the bathing of the bather (for example, a temperature of warm water in the bathtub body 4, the bathing time of the bather bathing in the bathtub body 4, and the heart rate of the bather). Here, the specific bathing is a bathing specified as when, such as bathing on what day of the month (and time of the day, if necessary).

In the present embodiment, the bather specifying unit 42 includes the pairing device 40 provided in the bath room R. The pairing device 40 can be connected to terminal equipment such as the bather's smartphone by wireless communication and can register pairing information as pairing (information on a possessor (bather) of the smartphone). The pairing device 40 is paired in advance with the terminal equipment such as the bather's smartphone and can individually recognize the bather's smartphone, or the like. Therefore, when the pairing device 40 is paired with terminal equipment such as a smartphone of a specified bather as a target at a comparatively close position of about several meters, the pairing device 40 can determine and specify that a possessor of the smartphone bathes as the specified bather. In the pairing device 40, information on a plurality of bathers is registered to correspond to a paired information terminal of each specified bather. The pairing device 40 can therefore recognize the registered terminal equipment and specify who the bather is. Note that the bather specifying unit 42 may implement a function of program of the terminal equipment such as the smartphone and may specify the bather by inputting, to the terminal equipment, who the bather is. Furthermore, the bather specifying unit 42 may implement a function of the program of the remote controller 9 and may specify the bather by inputting, to the remote controller 9, who the bather is. The "specifying the bather" in the present invention is not limited to specifying of personal information of the bather and includes, for example, determining which user is bathing among a plurality of users registered in the bathing information system 1 and the like in advance.

The temperature sensor 14 that measures the temperature of warm water stored in the bathtub body 4 provided in the bathtub apparatus 2, the negative electrode 5a, the positive electrode 5b and the like function as the information acquisition unit 44.

As shown in FIG. 3, the temperature sensor 14 acquires the temperature of warm water in the objective information A on the bathing of the bather, as the information acquisition unit 44. The temperature sensor 14 is provided on a supply port part 16 of warm water from the water heater to the bathtub. The temperature sensor 14 may be provided on a circulation flow channel to a water discharge part, or the like. The temperature sensor 14 is, for example, a thermistor.

The bathing information system 1 further includes the program 50 introduced in the terminal equipment 52 such as the bather's smartphone. For example, the program 50 is executed by the terminal equipment 52 to implement a predetermined function. The program 50 executes application software on the terminal equipment to implement the predetermined function.

The program 50 causes the terminal equipment 52 to function as a controller. The terminal equipment 52 is, for example, a portable information terminal such as the smartphone and may be other terminal equipment such as a desktop personal computer, a notebook personal computer, or a tablet terminal. The terminal equipment 52 includes a built-in CPU, memory, and others, and implements a predetermined control function based on the predetermined program 50 recorded in the memory or the like. The terminal equipment 52 is electrically connected to the pairing device 40 and the remote controller 9. These electrical connections may be connected entirely or partially by wireless communication such as infrared communication or another method. Therefore, the terminal equipment 52 may be located at a position physically away from the bathtub apparatus 2 via internet.

The terminal equipment 52 further includes a terminal equipment input section 52a that accepts input of subjective information B (see FIGS. 6 to 12B), and a terminal equipment display section 52b that displays a bathing history, history information, and the like, as an output device. The terminal equipment input section 52a accepts input on a liquid crystal screen by a touch panel or the like. The terminal equipment input section 52a may include a screen common to the terminal equipment display section 52b. The terminal equipment display section 52b displays information on a liquid crystal screen by a touch panel or the like. The terminal equipment display section 52b of the terminal equipment 52 can display history information or the like, as the output device. The history information and the like do not necessarily have to be displayed on both the terminal equipment display section 52b and the remote controller display section 9b and may be displayed only on either one of the sections. The subjective information is, for example, evaluation information based on feeling and sensitivity (subjectivity) of the bather, such as "satisfied", "fair", "dissatisfied", "good", "fair", "poor", and the like. Furthermore, as subjective information, descriptive impressions or the like based on the bather's feeling and sensitivity (subjectivity) may be used.

The program 50 is stored in a storage device such as the memory of the terminal equipment 52. The program 50 controls the terminal equipment 52 to store the subjective information B input by the bather in the terminal equipment 52 such as the smartphone that functions as a controller. The program 50 causes the terminal equipment 52 to store a bathing history obtained by associating the objective information A acquired by the information acquisition unit 44 regarding a specific bathing of a specified bather with the subjective information B input by the bather specified for the specific bathing. The program 50 causes the terminal equipment 52 to implement an output function of outputting history information C based on a plurality of bathing histories for the specified bather onto the output device such as the remote controller display section 9b of the remote controller 9 and/or the terminal equipment display section 52b of the terminal equipment 52. The program 50 may have a function of outputting history information onto the terminal equipment display section 52b of the terminal equipment 52 if the bather is not bathing. The program 50 may be stored on a bathtub apparatus controller 9a side and executed by the bathtub apparatus controller 9a. Furthermore, part of the program 50 may be stored on the bathtub apparatus controller 9a side and executed by the terminal equipment 52 and the bathtub apparatus controller 9a in cooperation. All or part of the program 50 may be disposed and stored on cloud, and the program 50 may be executed by the terminal equipment 52 or the like.

The objective information A is any of a warm water temperature of warm water in the bathtub, the bathing time, and the biological information of the bather, or a combination of all or part of the information. The warm water temperature of warm water in the bathtub and the bathing time in the objective information A objectively indicate a bathing environment. The biological information of the bather in the objective information A indicates reaction of a body of an objective bather according to the bathing environment.

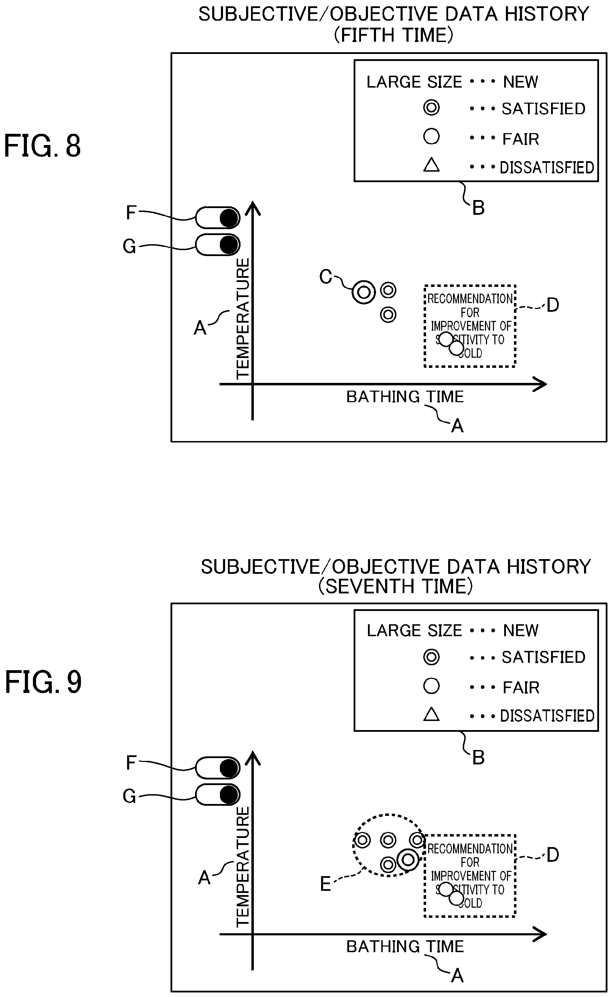

The program 50 further causes the terminal equipment 52 functioning as the controller to implement a function of outputting preset recommended information D together with the history information C to the remote controller display section 9b of the remote controller 9 and/or the terminal equipment display section 52b of the terminal equipment 52. The history information C includes recommended range information E (see FIG. 9) determined based on a plurality of bathing histories.

The output function implemented by the program 50 will be described in more detail.

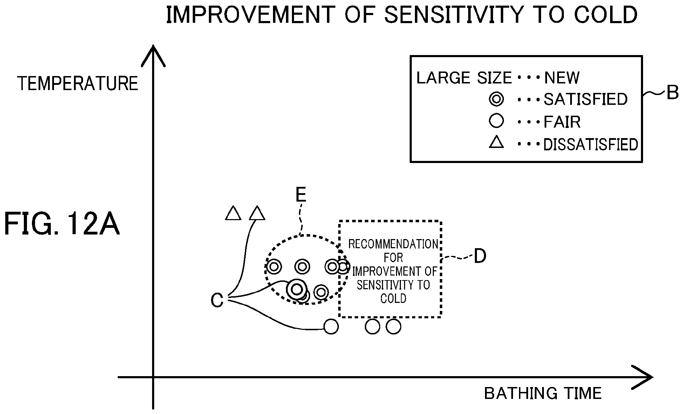

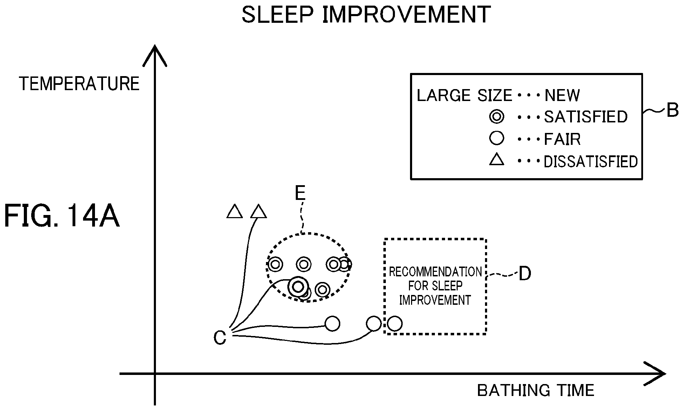

The output function includes a first output function and a second output function. The program 50 causes the terminal equipment 52 to store a first bathing history obtained by associating first objective information (bathing time and warm water temperature) selected for a first analysis application (for example, application of improvement of sensitivity to cold shown in FIG. 12A) from the objective information A with first subjective information input for the first analysis application from the subjective information B and causes the terminal equipment 52 to implement the first output function of outputting first history information based on a plurality of first bathing histories for a specified bather to the remote controller display section 9b or the like. The program 50 further causes the terminal equipment 52 to store a second bathing history obtained by associating second objective information (bathing time and warm water temperature) selected for a second analysis application (for example, a sleep improvement application shown in FIG. 14A) from the objective information A with second subjective information input for the second analysis application from the subjective information B and causes the terminal equipment 52 to implement the second output function of outputting second history information based on a plurality of second bathing histories for the specified bather to the remote controller display section 9b or the like. The program 50 further causes the terminal equipment 52 to implement a function of switching between the first output function and the second output function. The output function is not limited to two functions, the first output function and the second output function, and may include three or more output functions.

Next, an operation of the bathing information system according to the first embodiment of the present invention will be described with reference to FIGS. 6 to 11.

In FIGS. 6 to 11, the vertical axis indicates a temperature (°C) of warm water in the bathtub, and the horizontal axis indicates a bathing time (minutes). In addition, for the subjective information B input by the bather, new information is indicated with a comparatively large size mark (large size marks indicating satisfaction (double circle), fair (circle), and dissatisfaction (triangle)), and satisfaction is indicated with a double circle, fair is indicated with a circle, and dissatisfaction is indicated with a triangle. Furthermore, an upper portion of the ordinate provides a slide button F for turning on or off a function capable of determining whether to display the recommended range information E and a slide button G for turning on or off a function capable of determining whether to display the preset recommended information D. Reference signs A to G are shown for explanation and are not displayed on an actual screen (the same applies to FIGS. 7 to 14C below).

First, the specifying of the bather will be described. For example, during the specific bathing of the bather, the pairing device 40 recognizes the terminal equipment 52 such as the bather's smartphone, and the bathtub apparatus controller 9a of the remote controller 9 and the terminal equipment 52 specify who the bather is.

When the bather enters the bathtub, the information acquisition unit 44 acquires the objective information A. For example, when the negative electrode 5a, the positive electrode 5b, and the like start detecting an electrocardiogram waveform of the bather, the bathtub apparatus controller 9a can determine that the user has entered the bathtub, and starts measurement of the bathing time. Furthermore, the negative electrode 5a, the positive electrode 5b, and the like start measurement of the heart rate, and at the same time, the temperature sensor 14 starts measurement of the temperature of warm water in the bathtub.

When the bather exits the bathtub and finishes bathing in the bathtub, the information acquisition unit 44 ends the acquisition of the objective information A. For example, when the negative electrode 5a, the positive electrode 5b, and the like no longer detect the bather's electrocardiogram waveform, the bathtub apparatus controller 9a can determine that the user has finished bathing in the bathtub, and ends the measurement of the bathing time. Furthermore, the measurement of the heart rate by the negative electrode 5a, the positive electrode 5b, and the like ends, and at the same time, the measurement of the temperature of warm water in the bathtub by the temperature sensor 14 ends. Thus, the bather finishes specific bathing. The bathtub apparatus controller 9a acquires and stores an average temperature of the warm water during the bather's bathing as the temperature of warm water.

Thus, the bathtub apparatus controller 9a acquires and stores the objective information A acquired by the negative electrode 5a, the positive electrode 5b, and the temperature sensor 14. Furthermore, the terminal equipment 52 acquires and stores the objective information A from the bathtub apparatus controller 9a.

After the specific bathing, the bather inputs the subjective information B on this specific bathing into the terminal equipment input section 52a of the terminal equipment 52 in a period until the next bathing. For example, the bather inputs a subjective impression of improvement of sensitivity to cold for this specific bathing, for example, with elapse of several hours after the bathing. At the same time or at a different time, the bather can input subj ective impressions of sleep improvement and fatigue improvement for this specific bathing. In addition, the bather can also input a subjective impression of comfort of the bathing itself for this specific bathing immediately after the bathing, after elapse of a predetermined time, or the like. The program 50 controls the terminal equipment 52 to store the subjective information B input by the bather, in the terminal equipment 52 or the like.

As shown in FIG. 6, the program 50 causes the terminal equipment 52 to store a bathing history obtained by associating the obj ective information A (temperature of warm water in the bathtub and bathing time) acquired by the information acquisition unit 44 regarding the specific bathing of the specified bather (for example, first bathing (first measurement day)) with the subjective information B input by the specified bather for the specific bathing. The program 50 further causes the terminal equipment 52 to implement an output function of outputting history information C based on a plurality of bathing histories for the specified bather onto the remote controller display section 9b of the remote controller 9 and/or the terminal equipment display section 52b of the terminal equipment 52. Here, the program 50 can cause the terminal equipment 52 to output, to the output device, the history information C even based on a single bathing history. Even if recommended information based on a plurality of bathing histories is not displayed, the bather can comparatively early confirm comparison of the subjective information with the objective information and can use the comparison as a reference for future bathing. Thus, FIG. 6 illustrates the history information C based on the bathing history displayed on the terminal equipment 52.

As shown in FIG. 6, the program 50 further causes the terminal equipment 52 to implement a function of outputting the preset recommended information D together with the history information C to the output device. The recommended information D is illustrated as a region range in which it is assumed in advance that a general bather is likely to subjectively obtain a sense of satisfaction (indicated with the double circle) with respect to the objective information A such as the temperature of warm water and the bathing time. The recommended information D is shown, for example, in a region surrounded by a dashed line. The recommended information D can provide reference indicators such as a bathing time and temperature indicating that the general bather is likely to subjectively obtain the sense of satisfaction (double circle).

The bather can bathe referring to the history information C based on the bathing history up to the previous bathing in next and subsequent bathing (for example, second bathing, third bathing, and the like). For example, the third bathing will be described with reference to FIG. 7. First, for this specific bathing, the bathtub apparatus controller 9a of the remote controller 9 and the terminal equipment 52 specify who the bather is. Then, when the bather enters the bathtub, the information acquisition unit 44 acquires the objective information A. When the bather exits the bathtub and finishes bathing in the bathtub, the information acquisition unit 44 finishes acquiring the objective information A.

The bathtub apparatus controller 9a acquires and stores the objective information A acquired by the negative electrode 5a, the positive electrode 5b, and the temperature sensor 14. Furthermore, the terminal equipment 52 acquires and stores the objective information A from the bathtub apparatus controller 9a.

After this specific bathing, the bather inputs the subjective information B on this specific bathing into the terminal equipment input section 52a of the terminal equipment 52 in a period until the next bathing. The program 50 controls the terminal equipment 52 to store the subjective information B input by the bather in the terminal equipment 52.

The program 50 causes the terminal equipment 52 to store the bathing history obtained by associating the objective information A acquired by the information acquisition unit 44 regarding the specific bathing of the specified bather (for example, bathing on the third day) with the subjective information B input by the specified bather for the specific bathing. The program 50 further causes the terminal equipment 52 to implement an output function of outputting the history information C based on a plurality of bathing histories for the specified bather onto the remote controller display section 9b of the remote controller 9 and/or the terminal equipment display section 52b of the terminal equipment 52. Even if the number of bathing histories is comparatively small, the bather can comparatively early confirm the comparison of the subjective information B with the objective information A and can use the comparison as a reference for future bathing. Thus, FIG. 7 illustrates the history information C based on the bathing history displayed on the terminal equipment 52. For example, in the history information C, a circle mark is shown for new bathing (this bathing) because the bather inputs "fair" as subjective information for the new bathing (this bathing).

The program 50 causes the terminal equipment 52 to implement a function of outputting the preset recommended information D together with the history information C to the remote controller display section 9b of the remote controller 9 and/or the terminal equipment display section 52b of the terminal equipment 52. The bather can compare the history information C based on the plurality of bathing histories of the bather with the recommended information D and use the comparison as a reference in determining a bathing style.

As shown in FIG. 8, in a specific bathing (for example, fifth bathing), the information acquisition unit 44 similarly acquires the objective information A and the subjective information B of the bather is input into the terminal equipment input section 52a. The program 50 causes the terminal equipment 52 to store the bathing history obtained by associating the objective information A acquired by the information acquisition unit 44 regarding the specific bathing of the specified bather (for example, the bathing on the fifth day) with the subjective information B input by the specified bather for the specific bathing. The program 50 further causes the terminal equipment 52 to implement an output function of outputting the history information C based on a plurality of bathing histories for the specified bather onto the remote controller display section 9b of the remote controller 9 and/or the terminal equipment display section 52b of the terminal equipment 52.

In FIG. 9, in specific bathing (for example, seventh bathing or the like), the information acquisition unit 44 similarly acquires the objective information A and the subjective information B of the bather is input into the terminal equipment input section 52a. Thus, the program 50 causes the terminal equipment 52 to store a bathing history obtained by associating the objective information A acquired by the information acquisition unit 44 regarding the specific bathing of the specified bather (for example, the bathing on the seventh day) with the subjective information B input by the specified bather for the specific bathing. The program 50 further causes the terminal equipment 52 to implement an output function of outputting history information C based on a plurality of bathing histories for the specified bather onto the remote controller display section 9b of the remote controller 9 and/or the terminal equipment display section 52b of the terminal equipment 52.

In FIG. 9, the program 50 further causes the terminal equipment 52 to implement an output function of outputting the recommended range information E determined based on a plurality of bathing histories for the specified bather onto the remote controller display section 9b of the remote controller 9 and/or the terminal equipment display section 52b of the terminal equipment 52. The program 50 can display the recommended range information E (for example, displayed with a circle of a dashed line) including a region where, for example, satisfaction marks (double circles) gather in the subjective information B of the bather as the bathing history increases to multiple (for example, seven bathing histories in total). The recommended range information E can be used as a reference indicator of a region where the bather can easily obtain a sense of satisfaction.

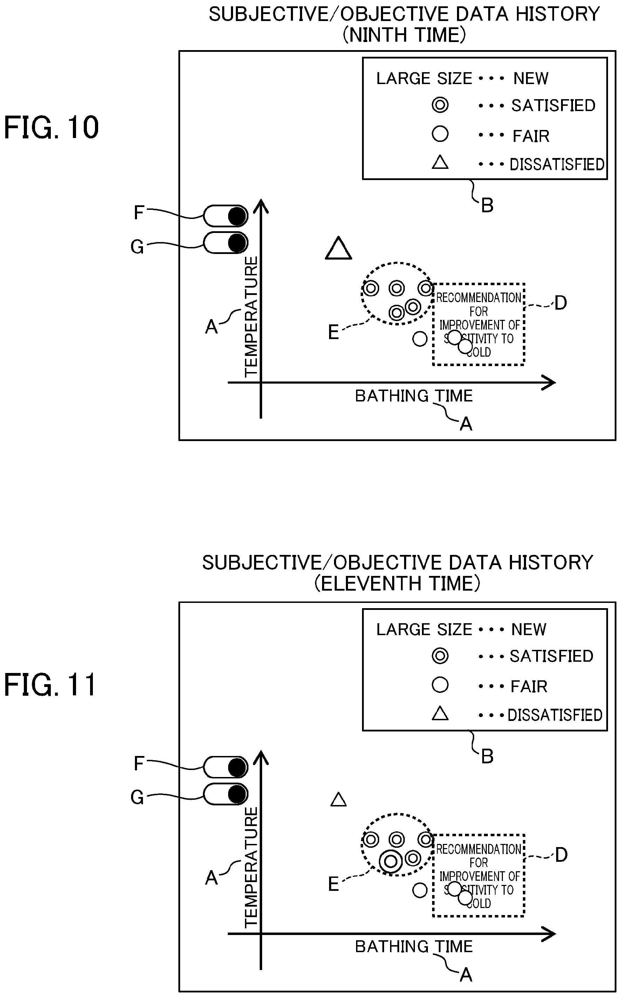

FIG. 10 shows that in specific bathing (for example, the ninth bathing or the like), the subjective information B (new and dissatisfaction (triangle)) of the bather is input into the terminal equipment input section 52a. The program 50 causes the terminal equipment 52 to store a bathing history obtained by associating the objective information A acquired by the information acquisition unit 44 regarding the specific bathing of the specified bather (for example, bathing on the seventh day) with the subjective information B input by the specified bather for the specific bathing. The program 50 further causes the terminal equipment 52 to implement an output function of outputting the history information C based on a plurality of bathing histories for the specified bather onto the remote controller display section 9b of the remote controller 9 and/or the terminal equipment display section 52b of the terminal equipment 52. As shown in FIG. 10, when the bather tries bathing at a temperature of warm water for a bathing time deviating from the recommended range information E and the recommended information D, the bather still does not obtain satisfactory results and input dissatisfaction (triangle). Consequently, the output function implemented by the program allows the bather to try bathing with new different condition settings while referring to the recommended range information E or the recommended information D, or bathing histories so far and to analyze and feedback resulting subjective information in relation to the histories so far.

FIG. 11 shows that in specific bathing (for example, the eleventh bathing or the like), the subjective information B (new and satisfaction (double circle)) of the bather is input into the terminal equipment input section 52a. When the bather tries bathing at a temperature of warm water for a bathing time within the recommended range information E, the bather still obtains satisfactory results and input satisfaction (double circle). Consequently, the output function implemented by the program 50 allows the bather to confirm a region where satisfaction is obtained while referring to the recommended range information E or recommended information D, or bathing histories so far and to analyze and feedback results in relation to the histories so far.

Next, the output function implemented by the program 50 for different analysis applications will be described in more detail.

FIG. 12A illustrates an output function of outputting the history information C based on a plurality of bathing histories for the application of improvement of sensitivity to cold onto the remote controller display section 9b of the remote controller 9 and/or the terminal equipment display section 52b of the terminal equipment 52. The bathing information system 1 can suggest, to each bather, a bathing method that is effective for a specific problem (improvement of sensitivity to cold, improvement of sleep, etc.) by such an output function. FIG. 12A illustrates the history information C, recommended range information E, recommended information D, and the like on a graph in the same manner as in FIGS. 6 to 11. Acquisition of the objective information A or subjective information B is the same as described above and therefore is not described. The program 50 causes the terminal equipment 52 to store, for example, a first bathing history obtained by associating first objective information (warm water temperature and bathing time) selected from the objective information A for the application of improvement of sensitivity to cold that is a first analysis application with first subjective information input for the first analysis application (bather's subjective information B on the application of improvement of sensitivity to cold) from the subjective information B and causes the terminal equipment 52 to implement a first output function of outputting first history information (history information C) based on a plurality of first bathing histories for a specified bather onto the remote controller display section 9b or the like. The first analysis application is illustrated as an example and can be changed to a second analysis application, a third analysis application, and the like described later. That is, the first output function is illustrated corresponding to the first analysis application for explanation and may be changed to a second output function, a third output function, and the like as well.

In FIG. 12B, the first output function of outputting the first history information (history information C) in a different form of table to the remote controller display section 9b is implemented based on the same data as the data shown in FIG. 12A. FIG. 12B displays a satisfaction level in three levels of satisfaction, fair, and dissatisfaction in a leftmost first column, date in a second column from the left, the temperature of warm water in a third column, the bathing time in a fourth column, and the heart rate in a fifth column. Therefore, FIG. 12B also illustrates an output function of outputting the first history information (history information C) based on a plurality of bathing histories for the application of improvement of sensitivity to cold onto the remote controller display section 9b of the remote controller 9 and/or the terminal equipment display section 52b of the terminal equipment 52. Thus, the program 50 can cause the terminal equipment 52 to output the history information C in the different form of table. FIG. 12B also shows that when any satisfaction level is not input yet (for example, in case of new (this time) non-input), the satisfaction level can be selected and input.

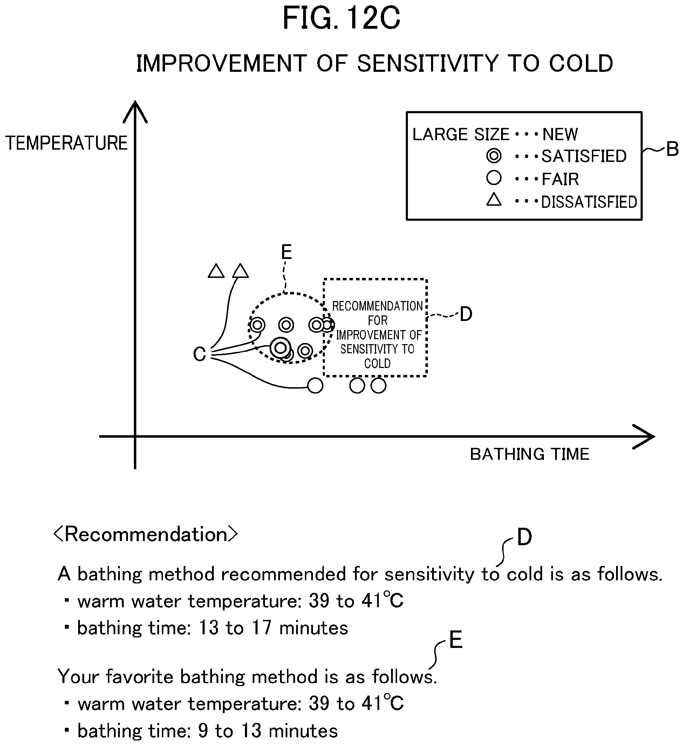

In FIG. 12C, the first output function of outputting, to the remote controller display section 9b, the first history information (history information C) in the graph of FIG. 12A and in sentences is implemented based on the same data as the data shown in FIGS. 12A and 12B. In FIG. 12C, a range of temperature of warm water and a range of bathing time in the preset recommended information D are described in a sentence, for example, "A bathing method recommended for sensitivity to cold is as follows: warm water temperature: 39 to 41°C; and bathing time: 13 to 17 minutes". The range of the recommended information D in such a sentence corresponds to the range of the recommended information D in the graph of FIG. 12C. Furthermore, in FIG. 12C, the range of temperature of warm water and the range of bathing time in the recommended range information E are described in a sentence, for example, "Your favorite bathing method is as follows: warm water temperature: 39 to 41°C; and bathing time: 9 to 13 minutes". The ranges of the recommended range information E in such a sentence correspond to the ranges of the recommended range information E in the graph of FIG. 12C. As such, since the program 50 can output the recommended information D, the recommended range information E, and the like in the form of sentences, the bather can more easily recognize the recommended information D or the like by the sentences.

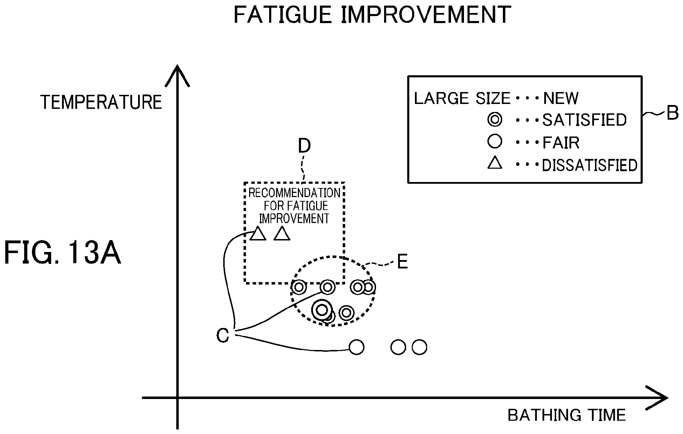

Next, FIG. 13A illustrates an output function of outputting the history information C based on a plurality of bathing histories onto the remote controller display section 9b of the remote controller 9 and/or the terminal equipment display section 52b of the terminal equipment 52 for a fatigue improvement application. FIG. 13A displays the history information, recommended range information E, recommended information D, and the like on a graph. The acquisition of the objective information A and subjective information B is the same as described above and is therefore not described.

The program 50 causes the terminal equipment 52 to store a second bathing history obtained by associating second objective information (warm water temperature and bathing time) selected from the objective information A for the fatigue improvement application that is a second analysis application with the second subjective information (subjective information of the bather regarding the fatigue improvement application) input for the second analysis application from the subjective information B and causes the terminal equipment 52 to implement the first output function of outputting second history information (history information C) based on a plurality of second bathing histories for a specified bather to the remote controller display section 9b or the like.

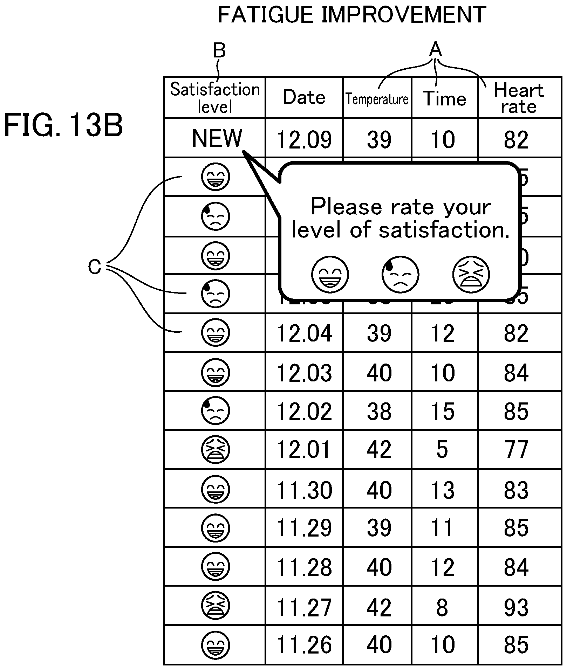

In FIG. 13B, a second output function of outputting, for example, the history information C that is the second history information in a different form of table to the remote controller display section 9b is implemented based on the same data as the data shown in FIG. 13A. FIG. 13B displays a satisfaction level as to whether a purpose of fatigue improvement is achieved at three levels of satisfaction, fair, and dissatisfaction in a leftmost first column, the date in a second column from the left, the temperature of warm water in a third column, the bathing time in a fourth column, and the heart rate in a fifth column. Therefore, FIG. 13B also illustrates an output function of outputting, for example, history information C that is the second history information based on a plurality of bathing histories onto the remote controller display section 9b of the remote controller 9 and/or the terminal equipment display section 52b of the terminal equipment 52 for the fatigue improvement application. As such, the program 50 can cause the terminal equipment 52 to output the history information C in a different form of table. FIG. 13B shows that when any satisfaction level is not input yet (for example, in case of new (this time) non-input), the satisfaction level can be selected and input.

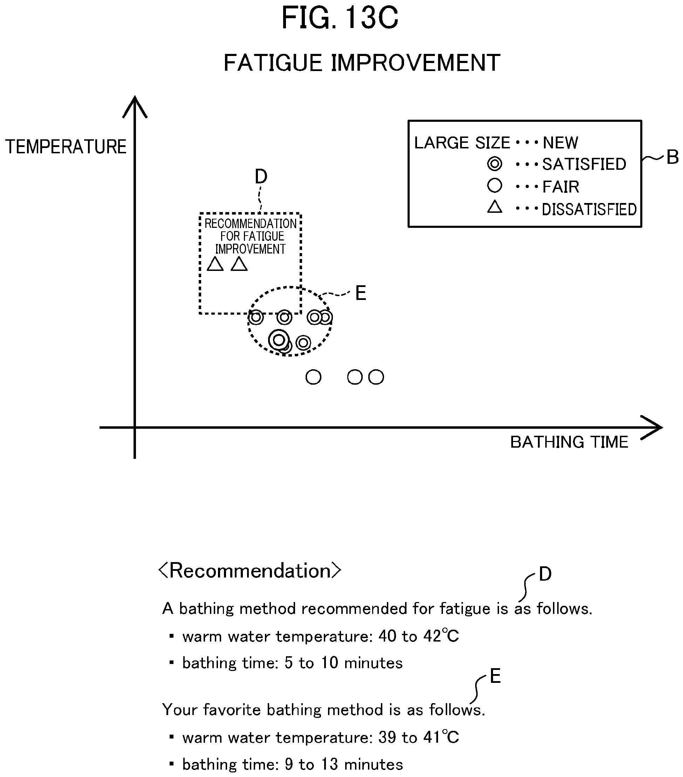

In FIG. 13C, the second output function of outputting the history information C in the graph of FIG. 13A and sentences to the remote controller display section 9b is implemented based on the same data as the data shown in FIGS. 13A and 13B. In FIG. 13C, a range of temperature of warm water and a range of bathing time in the preset recommended information D are described in a sentence, for example, "A bathing method recommended for fatigue is as follows: warm water temperature: 40 to 42°C; and bathing time: 5 to 10 minutes". The range of the recommended information D in such a sentence corresponds to the range of the recommended information D in the graph of FIG. 13C. Furthermore, in FIG. 13C, a range of temperature of warm water and a range of bathing time in the recommended range information E are described in a sentence, for example, "Your favorite bathing method is as follows: warm water temperature: 39 to 41°C; and bathing time: 9 to 13 minutes". The range of the recommended range information E in such a sentence corresponds to the range of the recommended range information E in the graph of FIG. 13C. As such, since the program can output the recommended information D, the recommended range information E, and the like in the form of sentences, the bather can more easily recognize the recommended information D and the like by the sentences.

Next, FIG. 14A illustrates an output function of outputting the history information C based on a plurality of bathing histories for the sleep improvement application to the remote controller display section 9b of the remote controller 9 and/or the terminal equipment display section 52b of the terminal equipment 52. In FIG. 14A, the history information C, recommended range information E, recommended information D, and the like are displayed on a graph. The acquisition of the objective information A and subjective information B is the same as described above and therefore is not described. The program 50 causes the terminal equipment 52 to store a third bathing history obtained by associating, for example, third objective information (temperature of warm water and bathing time) selected from the objective information A for the sleep improvement application that is a third analysis application with third subjective information (bather's subjective information on the fatigue improvement application) input from the subjective information B for the sleep improvement application, and causes the terminal equipment 52 to implement a third output function of outputting, to the remote controller display section 9b, for example, the history information C that is third history information based on a plurality of third bathing histories for a specified bather.

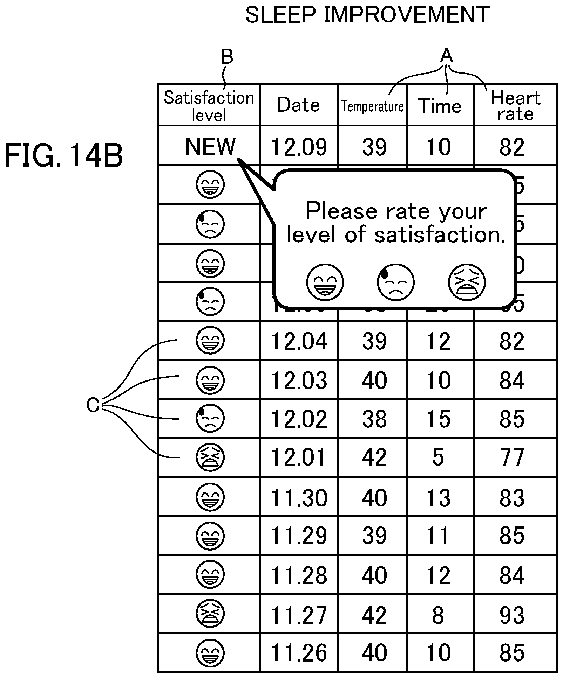

In FIG. 14B, the third output function of outputting, for example, the history information C that is the third history information in a different form of table to the remote controller display section 9b is implemented based on the same data as the data shown in FIG. 14A. FIG. 14B displays a satisfaction level as to whether a purpose of fatigue improvement is achieved in three levels of satisfaction, fair, and dissatisfaction in a leftmost first column, date in a second column from the left, the temperature of warm water in a third column, the bathing time in a fourth column, and the heart rate in a fifth column. Therefore, FIG. 14B also illustrates an output function of outputting, for example, the history information C that is the third history information based on a plurality of bathing histories for the sleep improvement application to the remote controller display section 9b of the remote controller 9 and/or the terminal equipment display section 52b of the terminal equipment 52. Thus, the program 50 can cause the terminal equipment 52 to output the history information in the different form of table. FIG. 14B shows that when any satisfaction level is not input yet (for example, in case of new (this time) non-input), the satisfaction level can be selected and input.

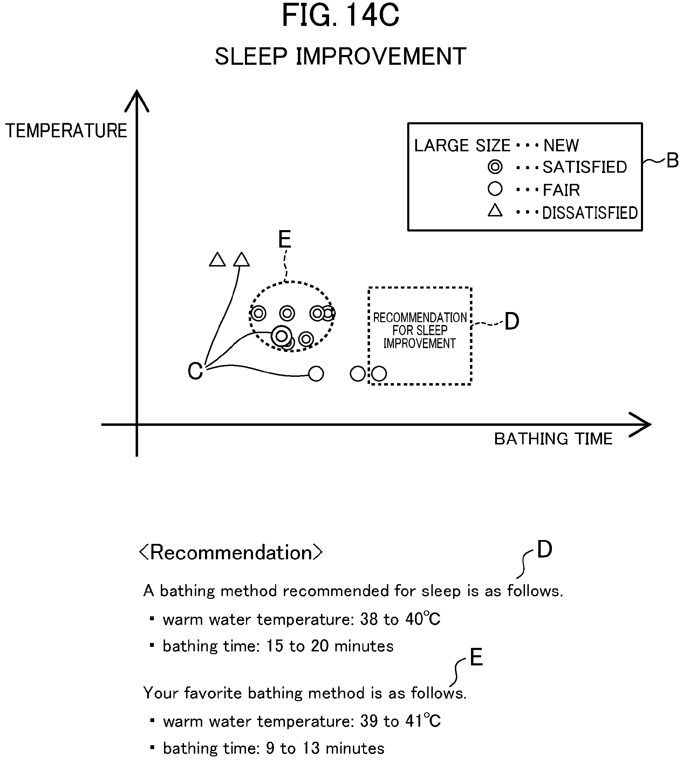

In FIG. 14C, the third output function of outputting the history information C in the graph of FIG. 14A and a different form of sentence to the remote controller display section 9b is implemented based on the same data as the data shown in FIGS. 14A and 14B. In FIG. 14C, a range of temperature of warm water and a range of bathing time in the preset recommended information D are described in a sentence, for example, "A bathing method recommended for good sleep is as follows: warm water temperature: 38 to 40°C; and bathing time: 15 to 20 minutes". The range of the recommended information D in such a sentence corresponds to the range of the recommended information D in the graph of FIG. 14C. Furthermore, in FIG. 14C, a range of temperature of warm water and a range of bathing time in the recommended range information E are described in a sentence, for example, "Your favorite bathing method is as follows: warm water temperature: 39 to 41°C; and bathing time: 9 to 13 minutes". The range of the recommended range information E in such a sentence corresponds to the range of the recommended range information E in the graph of FIG. 14C. Since the program can output the recommended information D, the recommended range information E, and the like in the form of sentence, the bather can more easily recognize the recommended information D and the like by the sentence.

In the first embodiment of the present invention including this configuration, the terminal equipment 52 includes the bathing history obtained by associating the objective information A acquired by the information acquisition unit 44 regarding the specific bathing of the specified bather with the subjective information B input by the specified bather for the specific bathing, and outputs, to the remote controller 9, the history information C based on the plurality of bathing histories for the specified bather. Consequently, the bather can confirm the objective information A in association with the subjective information B by the history information C based on the plurality of bathing histories. The present embodiment can help the bather to know a bathing method suitable for the bather by comparing the subjective information B with the objective information A in the plurality of bathing histories and can make it easier for the bather to achieve more comfortable bathing.

In the first embodiment of the present invention including this configuration, the objective information A includes the warm water temperature and bathing time indicating a bathing environment, and the bather can confirm the warm water temperature and bathing time in association with the subjective information B by the history information C based on the plurality of bathing histories. The present embodiment can help the bather to know the bathing method suitable for the bather by comparing the subjective information B of the bather with the warm water temperature and bathing time in the plurality of bathing histories and can make it easier for the bather to achieve more comfortable bathing.

In the first embodiment of the present invention including this configuration, the objective information A includes the biological information of the bather, and the bather can confirm the biological information of the bather that is a reaction of bather's body in association with the subjective information B by the history information C based on the plurality of bathing histories. The present embodiment can help the bather to know the bathing method suitable for the bather by comparing the bather's subjective information B with the biological information of the bather that is the reaction of the bather's body in the plurality of bathing histories and can make it easier for the bather to achieve more comfortable bathing.

In the first embodiment of the present invention including the above configuration, the terminal equipment 52 performs the recommended information output function of outputting, to the remote controller 9, the preset recommended information together with the history information C. Consequently, the bather can compare the preset recommended information with the history information C of the bather, can be helped in adjusting the bathing method of the bather, and can try the bathing method within the range of the preset recommended information.

In the first embodiment of the present invention including the above configuration, the history information C includes the recommended range information E determined based on the plurality of bathing histories. Consequently, the present embodiment can help the bather to know the bathing method more suitable for the bather while comparing the subjective information B of the bather with the objective information A by the recommended range information E and can further make it easier for the bather to achieve more comfortable bathing.

In the first embodiment of the present invention including the above configuration, the terminal equipment 52 can output the first history information for the first analysis application to the remote controller 9 by the first output function, can output the second history information for the second analysis application to the remote controller 9 by the second output function and can further switch between the first output function and the second output function by a switching function. Consequently, the present embodiment can help the bather to know the bathing method more suitable for the bather while switching and comparing the history information C for different analysis applications by the switching function and can further make it easier for the bather to achieve more comfortable bathing.

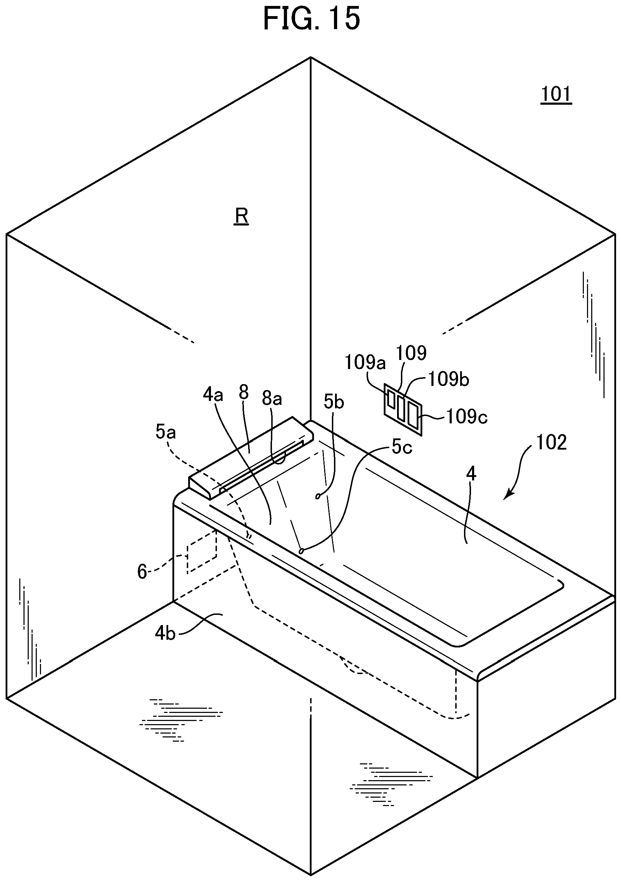

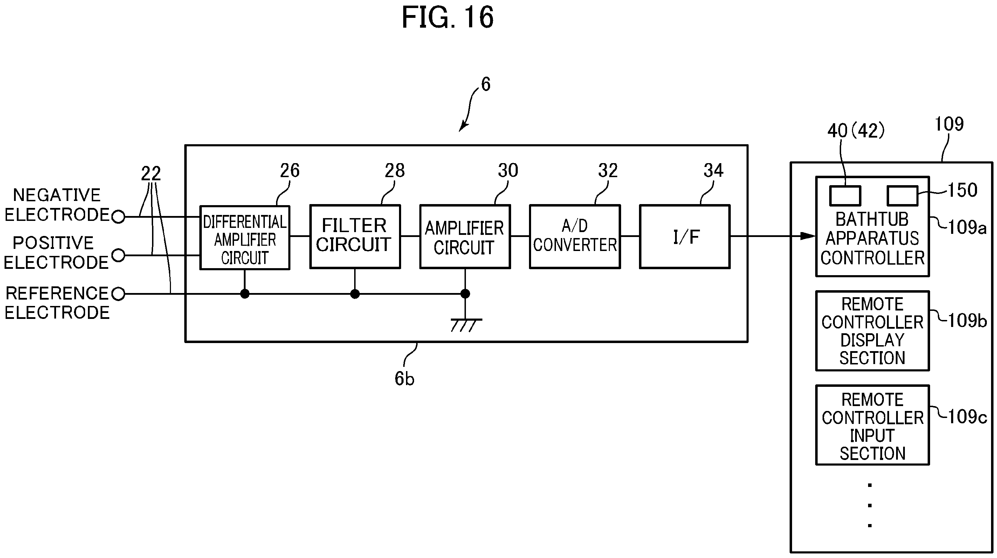

Next, a bathing information system according to a second embodiment of the present invention will be described with reference to FIGS. 15 and 16. The second embodiment is an example in which the bathing information system includes a bathtub apparatus so that a remote controller 109 of the second embodiment implements the function of the terminal equipment 52, in place of the terminal equipment 52 in the first embodiment. FIG. 15 is a perspective view showing an entire bath room in which the bathing information system according to the second embodiment of the present invention is installed. FIG. 16 is a diagram showing a schematic configuration of a signal processing device and the remote controller of the bathing information system according to the second embodiment of the present invention.

A bathing information system 101 according to the second embodiment includes a similar basic structure to the bathing information system 1 according to the first embodiment described above. Therefore, only differences of the second embodiment of the present invention from the first embodiment will be described, and similar parts are denoted with the same reference numerals in the drawing and are not described.

As shown in FIG. 15, the bathing information system 101 of the second embodiment of the present invention acquires information on a bather bathing in a bathtub. The bathing information system 101 includes a bathtub apparatus 102 installed in a bath room R. The bathtub apparatus 102 includes the remote controller 109 attached to a wall surface of the bath room R.

Next, a configuration of a signal processing device 6 and the remote controller 109 will be described with reference to FIG. 16.

The remote controller 109 in the second embodiment is configured to execute the function of the terminal equipment 52 by itself, in place of the terminal equipment 52 in the first embodiment. That is, the bathtub apparatus 102 implements a predetermined output function.

The remote controller 109 has a control function capable of controlling supply of warm water from a water heater 18 to the bathtub apparatus 2 and can operate an electrocardiogram signal detecting function of the bathtub apparatus 2 and a water discharge device 8. The remote controller 109 includes a bathtub apparatus controller 109a capable of controlling the supply of warm water to the bathtub apparatus 2, a remote controller display section 109b that displays a bathing history, history information, and the like, as an output device, and a remote controller input section 109c that accepts input of a command to the bathtub apparatus controller 109a.

The bathtub apparatus controller 109a controls equipment including a built-in CPU, memory, and the like and connected such that the equipment can control the supply of warm water from the water heater 18 to the bathtub apparatus 102, and the bathtub apparatus 102 based on predetermined control program recorded in the memory or the like. The bathtub apparatus controller 109a is electrically connected to the water heater 18, the signal processing device 6, a pump 8e of a water discharge device 8, a temperature sensor 14, the remote controller display section 109b, the remote controller input section 109c, and others. These electrical connections may be performed by wireless communication or the like. The bathtub apparatus controller 109a may be electrically connected to the temperature sensor 14 or the like by wireless communication or the like.

The bathtub apparatus controller 109a acquires and records signals sent via the signal processing device 6 from the negative electrode 5a, the positive electrode 5b and the like. Therefore, the negative electrode 5a, the positive electrode 5b and the like acquire a heart rate and bathing time from objective information A on the bathing of the bather. The bathtub apparatus controller 109a calculates the heart rate of the bather based on the signal sent from the signal processing device 6, as a heart rate conversion unit.

Furthermore, the bathtub apparatus controller 109a acquires and records a temperature of warm water in a bathtub body 4 from the temperature sensor 14.

The remote controller display section 109b displays information received from the bathtub apparatus controller 109a. The remote controller display section 109b forms a display section of a screen of a monitor, a touch panel or the like disposed in the bath room R. The remote controller display section 109b may further include a display device disposed outside the bath room R (the same applies to the remote controller input section 109c described later). The remote controller display section 109b has a function of displaying bathing history, history information, and the like, as an output device.

The remote controller input section 109c is configured to accept input of a command to control the bathtub apparatus controller 109a on a screen by a touch panel or the like. The remote controller input section 109c has a function of accepting input of subjective information B.

In the present embodiment, a bather specifying unit 42 of the bathtub apparatus 2 corresponds to a pairing device 40 implemented as a function of program of the remote controller 109. The bather specifying unit 42 specifies a bather by input of who the bather is to the remote controller 109.

The bathing information system 101 further includes program 150 (see FIG. 16) introduced into the bathtub apparatus controller 109a. For example, the program 150 is executed by the bathtub apparatus controller 109a to implement a predetermined function. The program 150 in the second embodiment has an output function of outputting history information C or the like onto the remote controller display section 109b in the same way as the output function of outputting the history information C or the like onto the remote controller display section 9b by the program 50 in the first embodiment. Therefore, the program 150 causes the bathtub apparatus controller 109a to function as a controller.

The program 150 is stored in a storage device such as the memory of the bathtub apparatus controller 109a. The program 150 controls the bathtub apparatus controller 109a to store the subjective information B input by the bather in the bathtub apparatus controller 109a functioning as the controller. Therefore, the bathtub apparatus controller 109a stores the objective information A and subjective information B. The program 150 causes the bathtub apparatus controller 109a to store a bathing history obtained by associating the objective information A acquired by an information acquisition unit 44 regarding specific bathing of the specified bather with the subjective information B input by the specified bather for the specific bathing. Therefore, the bathtub apparatus controller 109a stores a bathing history obtained by associating the objective information A acquired by the information acquisition unit 44 regarding the bather specified for specific bathing with the subjective information B input by the bather specified for the specific bathing. The program 150 causes the bathtub apparatus controller 109a to implement an output function of outputting the history information C based on a plurality of bathing histories for the specified bather onto the remote controller display section 109b that is the output device. Therefore, the bathtub apparatus controller 109a displays the history information C based on the plurality of bathing histories for the specified bather on the remote controller display section 109b that is the output device. All or part of the program 150 may be disposed and stored on cloud, and the program 150 may be read out of the cloud and executed by the bathtub apparatus controller 109a.

The program 150 further causes the bathtub apparatus controller 109a functioning as the controller to implement a function of outputting preset recommended information D together with the history information C to the remote controller display section 109b.

The output function implemented by the program 150 will be described in more detail.

The output function includes a first output function and a second output function. The program 150 causes the bathtub apparatus controller 109a to store a first bathing history obtained by associating first objective information selected from the objective information A for a first analysis application (for example, application for improving sensitivity to cold as shown in FIG. 12A) with first subjective information input for the first analysis application from the subjective information B and causes the bathtub apparatus controller 109a to implement the first output function of outputting first history information based on a plurality of first bathing histories for a specified bather to the remote controller display section 109b. The program 150 further causes the bathtub apparatus controller 109a to store a second bathing history obtained by associating second objective information selected from the objective information A for a second analysis application (for example, a sleep improvement application shown in FIG. 14A) with second subjective information input for the second analysis application from the subjective information B and causes the bathtub apparatus controller 109a to implement the second output function of outputting second history information based on a plurality of second bathing histories for the specified bather to the remote controller display section 109b. The program 150 further causes the bathtub apparatus controller 109a to implement a function of switching between the first output function and the second output function. The output function implemented by the program 150 can be implemented with respect to the output function implemented by the program 50 of the other first embodiment.

In the second embodiment of the present invention including the above configuration, the controller stores the bathing history obtained by associating the objective information A acquired by the information acquisition unit for the specific bathing of the specified bather with the subjective information B input in the input unit for the bather specified for the specific bathing, and displays, on the remote controller display section 109b, the history information C based on the plurality of bathing histories for the specified bather. Consequently, the bather can confirm the objective information A in association with the subjective information B by the history information C based on the plurality of bathing histories. The present embodiment can help the bather to know the bathing method suitable for the bather by comparing the subjective information B with the objective information A in the plurality of bathing histories and can make it easier for the bather to achieve more comfortable bathing.

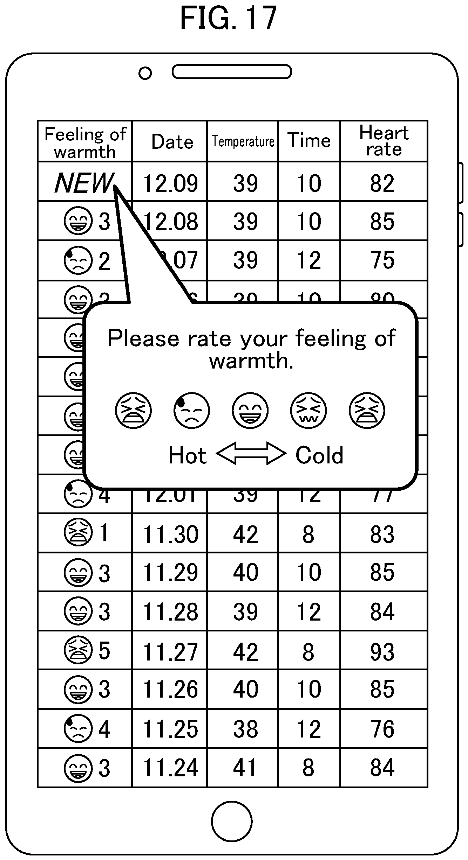

Next, a bathing information system according to a third embodiment of the present invention will be described with reference to FIGS. 17 to 24.

In the bathing information system according to the first embodiment of the present invention described above, a pairing device 40, which is a bather specifying unit, is communicated with, and connected to bather's smartphone (terminal equipment 52), to specify the bather. Furthermore, a negative electrode 5a, a positive electrode 5b and a temperature sensor 14 constituting an information acquisition unit 44 provided in a bathtub acquire a temperature of bathing warm water and the bathing time as objective information on bathing of a specified bather. After exiting the bathtub, the bather inputs subjective information on the bathing into the smartphone. For example, the bather selects a felt effect of the bathing on the improvement of sensitivity to cold as an analysis application from three subjectivities of "satisfied", "fair", and "dissatisfied" and inputs the selected subjectivity as subjective information (FIG. 12B, etc.).

Thus, the objective information and subjective information on the bathing are collected, associated, and stored as a bathing history. Then, the stored bathing history is displayed on the bather's smartphone. Additionally, preset recommended information indicating conditions recommended for bathing to improve the sensitivity to cold, for example, is also displayed (FIG. 12A, etc.).

In contrast, the bathing information system according to the third embodiment of the present invention has, in addition to the above functions, a function of collecting the objective information and subjective information for bathing of a specified bather and presuming subjective information that would correspond to a value of objective information (for example, warm water temperature, bathing time) to which any subjective information has not been tied by the bather. Furthermore, the presumed subjective information is output to a smartphone (terminal equipment 52) or a remote controller 9 that is an output device. The configuration of the bathing information system according to the third embodiment of the present invention is the same as the first embodiment described above, and the functions implemented by program are different from those in the first embodiment described above.

Therefore, in the following, only differences of the third embodiment of the present invention from the first embodiment will be described, and description of a similar configuration, operation, and effect will be omitted.

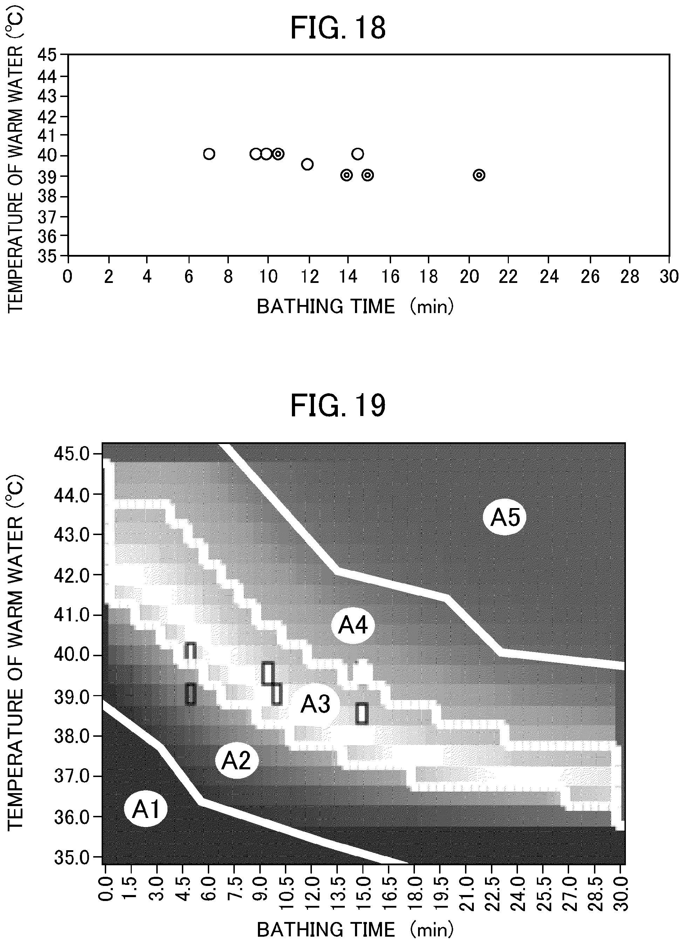

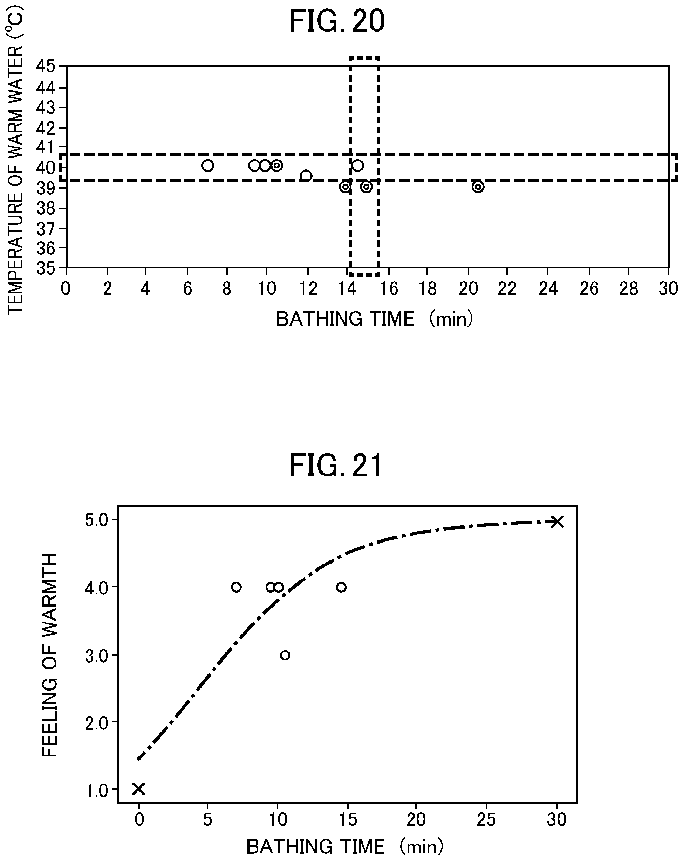

FIG. 17 is a diagram illustrating an example of a bathing history acquired in the bathing information system of the third embodiment of the present invention. FIG. 18 is a graph illustrating an example of a bathing history representing subjective information corresponding to a temperature of warm water and a bathing time. FIG. 19 is a diagram illustrating an example of a heat map representing subjective information corresponding to a temperature of warm water and bathing time presumed based on the bathing history. FIGS. 20 to 22 are diagrams illustrating an example of a procedure of presuming subjective information corresponding to a value of objective information to which any subjective information is not tied, based on the acquired bathing history.

In the example shown in FIG. 17, the temperature of warm water in the bathtub, the bathing time, and the heart rate of the bather after exiting the bathtub are acquired as objective information at each bathing time. For each bathing, the bather inputs subj ective information. The example shown in FIG. 17 is configured such that as the subjective information, bather's "feeling of warmth" is input in five levels of "too hot", "slightly hot", "comfortable", "slightly cold", and "too cold". That is, in the present embodiment, an analysis application of the bathing information system is "feeling of warmth".

FIG. 18 is a graph of plotted subjective information of "feeling of warmth" input by the bather corresponding to the temperature of warm water in the bathtub and the bathing time, which are objective information. Specifically, FIG. 18 plots each bathing on the graph with the bathing time on a horizontal axis and the temperature of warm water on a vertical axis, shows "comfortable" "feeling of warmth" corresponding to the bathing with a double circle, and shows "slightly hot" with a circle.

As shown in FIG. 18, the value of the objective information stored as the bathing history is only in a numerical range of small part of assumed warm water temperature and bathing time. Consequently, any subjective information of the bather is not tied to the value of the objective information that is not stored in the bathing history, and during bathing with the value of the objective information to which any subjective information is not tied, it is unknown, from the bathing history alone, whether the bather can obtain a comfortable "feeling of warmth".

For example, in the example shown in FIG. 18, any plot point is not present in regions where the temperature of the warm water is higher than about 40.5°C and lower than about 38°C, and in the regions, any value (temperature) of the objective information tied to the subjective information is not present. Furthermore, in the example shown in FIG. 18, any plot point is not present in regions where the bathing time is shorter than about seven minutes and longer than about 21 minutes, and any value (bathing time) of the objective information tied to the subjective information is not present. Consequently, the subjective information corresponding to the value of the objective information to which any subjective information is not tied is unknown. For example, in the example shown in FIG. 18, it is not possible to know, only from the bathing history, the temperature of bathing warm water at which the bather can obtain the comfortable "feeling of warmth" in bathing if the bathing time is shorter than about 7 minutes.

In contrast, according to the bathing information system of the present embodiment, subjective information that would correspond to the value of the objective information to which any subjective information has not yet been tied is presumed based on objective information on at least one bathing and subjective information tied to the value of the objective information. That is, in the bathing information system of the present embodiment, the heat map shown in FIG. 19 is presumed based on the bathing history shown in FIG. 18.

A heat map shown in FIG. 19 shows a region where the bather will have a comfortable "feeling of warmth" (subjective information of "feeling of warmth" is input as "comfortable") on a graph with the bathing time on the horizontal axis and the temperature of warm water on the vertical axis. That is, the heat map shown in FIG. 19 shows a region where the bather will feel "too cold" as A1, a region of "slightly cold" as A2, a region of "comfortable" as A3, a region of "slightly hot" as A4, and a region of "too hot" as A5. By use of this heat map, for example, it is possible to know the bathing time for which the bather is presumed to have the "comfortable" "feeling of warmth" from the temperature of warm water that the bather is to bathe.

Next, an example of a procedure for presuming the heat map shown in FIG. 19 will be described with reference to FIGS. 20 to 22.

First, as shown in FIG. 20, a plot point in the vicinity of the temperature of warm water at 40°C (region in a horizontal rectangle in FIG. 20) is extracted from the objective information of the bathing history stored as a set of the temperature of warm water and the bathing time.

Next, as shown in FIG. 21, plot points (circles) extracted from FIG. 20 are plotted on a graph with the "bathing time" on the horizontal axis and "the feeling of warmth" on the vertical axis ("too cold" = "1" to "too hot" = "5"). Here, in the stored bathing history, there is no plot point (circle) at which the bathing time is five minutes or less or no plot point (circle) at which the bathing time is 20 minutes or more. Then, the plot points (circles) on the graph shown in FIG. 21 are concentrated for the bathing time of about 10 to 15 minutes.

However, it is generally assumed that the bathing time of about 0 minutes is evaluated as "too cold" in the subjective information on the "feeling of warmth" and that the bathing time of about 30 minutes is evaluated as "too hot" in the subjective information, and hence plot points (crosses) representing such evaluations are added into the graph of FIG. 21. Based on the plot points (circles) and the plot points (crosses) on FIG. 21 thus obtained, interpolation by logistic equations can be performed to obtain a regression curve shown with a dashed chain line in FIG. 21. Consequently, when the temperature of warm water is in the vicinity of 40°C, a presumed value of subjective information (feeling of warmth) corresponding to each bathing time is calculated. Similarly, for another temperature band, a presumed value of subjective information (feeling of warmth) corresponding to each bathing time is also calculated.

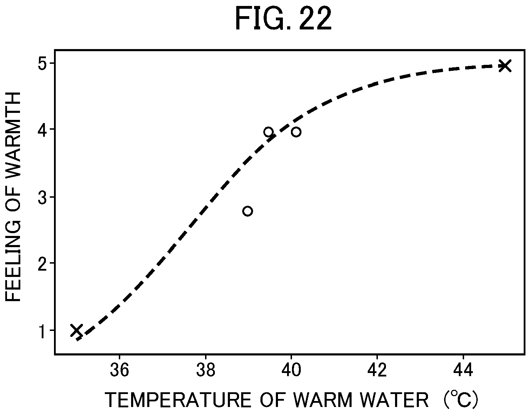

Next, subjective information corresponding to each temperature of warm water is presumed based on the presumed value of subjective information (feeling of warmth) corresponding to each bathing time obtained as shown in FIG. 21. FIG. 22 is a graph with "temperature of warm water" on the horizontal axis and "feeling of warmth" on the vertical axis, and the "feeling of warmth" in the vicinity of the bathing time of 15 minutes is plotted as a plot point (circle) (region in a vertical rectangle in FIG. 20). As shown in FIG. 22, in the temperature of warm water interpolated as shown in FIG. 21, there is no plot point (circle) at a temperature of 36°C or less or no plot point (circle) at a temperature of warm water of 44°C or more. Then, plot points (circles) on the graph shown in FIG. 22 are concentrated between temperatures of warm water of about 39 and 41°C.

However, it is generally assumed that the temperature of warm water of about 35°C is evaluated as "too cold" in the subjective information on the "feeling of warmth" and that the temperature of warm water of about 45 °C is evaluated as "too hot" in the subjective information, and hence plot points (crosses) representing such evaluations are added into the graph of FIG. 22. Based on the plot points (circles) and the plot points (crosses) on FIG. 22 thus obtained, interpolation by logistic equations can be performed to obtain a regression curve shown with a dashed line in FIG. 22. Consequently, when the bathing time is about 15 minutes, a presumed value of subjective information (feeling of warmth) corresponding to each temperature of warm water is calculated.

Furthermore, as described above, FIG. 22 shows presumption of the "feeling of warmth" in the vicinity of the bathing time of 15 minutes with respect to each temperature of warm water. Similarly, for another band of the bathing time, a presumed value of the subjective information (feeling of warmth) is also calculated corresponding to each temperature of warm water. Consequently, the presumed values of subjective information (feeling of warmth) corresponding to each bathing time and each temperature of warm water can be calculated, to obtain the heat map shown in FIG. 19.

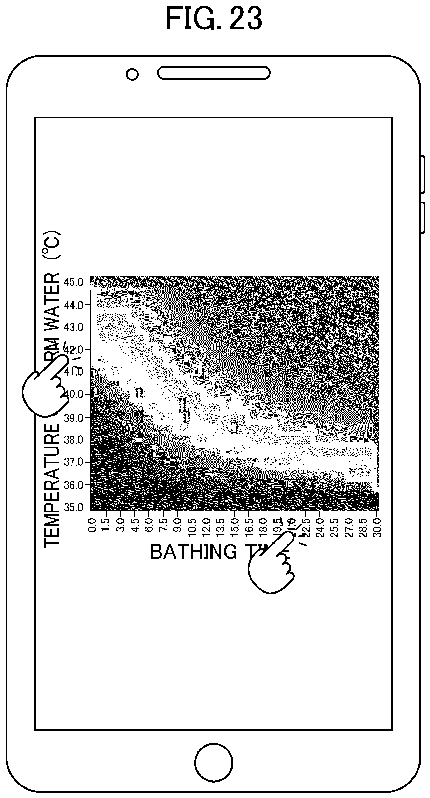

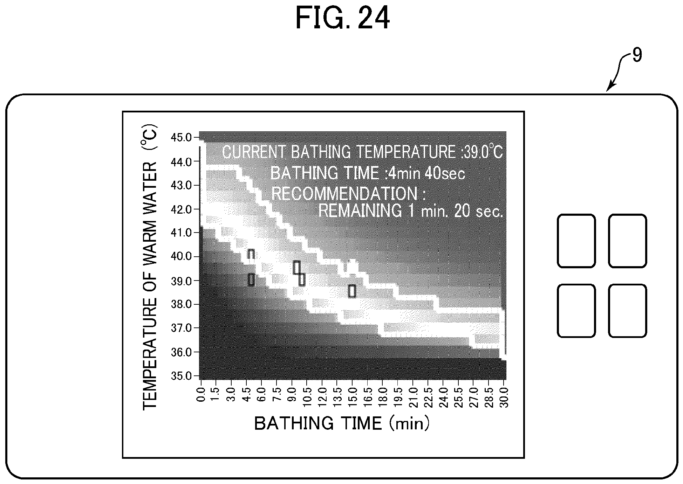

Next, an operation of the bathing information system according to the third embodiment of the present invention will be described with reference to FIGS. 23 and 24.

FIG. 23 is a view illustrating an example of a screen displayed on a smartphone (terminal equipment 52) before the bather starts bathing. FIG. 24 is a view illustrating an example of a screen displayed on a remote controller 9 while the bather is bathing.

As shown in FIG. 23, in the bathing information system of the present embodiment, when the bather activates a predetermined application program on a smartphone before bathing, a heat map presumed based on the stored bathing history is displayed. Here, when a set temperature of the bathtub apparatus to enter is set to, for example, 41°C, and warm water at 41°C is stored in the bathtub, the bather taps around 41°C on the vertical axis of the heat map. Then, even if the bather has never bathed in warm water at 41°C in the past and when the temperature of warm water is 41°C, a bathing time for which the subjective information on "feeling of warmth" is presumed to be "comfortable" is calculated. That is, the bathing time is calculated based on the heat map by the program activated on the smartphone. The calculated bathing time is, for example, displayed as "recommended bathing time is from about six minutes to nine minutes" (not shown) below the heat map on the screen of the smartphone.

In contrast, if the bather wishes to "bathe for about 20 minutes today" before bathing, the bather taps around 20 minutes on the horizontal axis of the heat map. Then, even if the bather has never bathed for 20 minutes in the past and when the bathing time is 20 minutes, the temperature of warm water at which subjective information on "feeling of warmth" is presumed to be "comfortable" is calculated based on the heat map. The calculated temperature of warm water is displayed, for example, as "recommended temperature of warm water is from about 37.5°C to 38.5°C" (not shown) below the heat map on the screen of the smartphone. Based on this display, the bather may change the set temperature of the bathtub apparatus and obtain comfortable feeling of warmth in the desired bathing time. Alternatively, the bathing information system may be configured to automatically change the set temperature of the bathtub apparatus based on the recommended temperature of warm water.

In contrast, when the bather bathes in the bathtub, a screen shown in FIG. 24 is displayed on the remote controller 9. That is, as shown in FIG. 24, a display section of the remote controller 9 displays the temperature of warm water currently stored in the bathtub, an elapsed time after bathing (elapsed time from when the bather enters the bathtub up to the present), and a remaining time until time to exit the bathtub recommended for obtaining the comfortable "feeling of warmth". The example shown in FIG. 24 shows that the temperature of warm water stored in the bathtub is 39°C, 4 minutes and 40 seconds elapses after the bather enters the bathtub, and the bather can obtain the comfortable "feeling of warmth" after bathing further for 1 minute and 20 seconds. Consequently, the bather can easily recognize when to exit the bathtub to obtain the comfortable "feeling of warmth" by looking at the screen of the remote controller 9 during bathing.

Furthermore, in the third embodiment described above, the temperature of warm water and the bathing time are acquired as the objective information, and in place of or in addition to the temperature of warm water and/or the bathing time, biological information such as a heart rate can be acquired as objective information. Furthermore, in the third embodiment described above, the bather inputs the "feeling of warmth" as the subjective information, and the present invention may be configured to input "improvement of sensitivity to cold", "fatigue improvement", "sleep improvement", "perspiration" or the like as subjective information in place of the "feeling of warmth".

According to the bathing information system of the third embodiment of the present invention, the program presumes the subjective information corresponding to the value of the objective information to which any subjective information is not tied (FIG. 19), and it is therefore possible to presume the subjective information indicating, for example, whether the bather feels warmed sufficiently or whether the bather perspires, for the values of objective information including bathing conditions the bather has not experienced yet and biological information of the bather. As a result, it is possible to recommend the value of objective information that may satisfy the bather even for the value of objective information in a region that is not experienced by the bather and to provide comfortable bathing.

According to the bathing information system of the present embodiment, it is possible to presume how the bather feels (subjective information) for the temperature of warm water and bathing time, for which the bather has not input any subjective information, based on the temperature of warm water in the bathtub and the bathing time, which are objective information, and based on the subjective information corresponding to the objective information. Consequently, for example, even if the bather is bathing in warm water at a temperature for which the bather has not input any subjective information, it is possible to presume how much bathing time is taken to obtain sufficient feeling of warmth (FIGS. 23 and 24) and to recommend bathing conditions that can satisfy the bather.

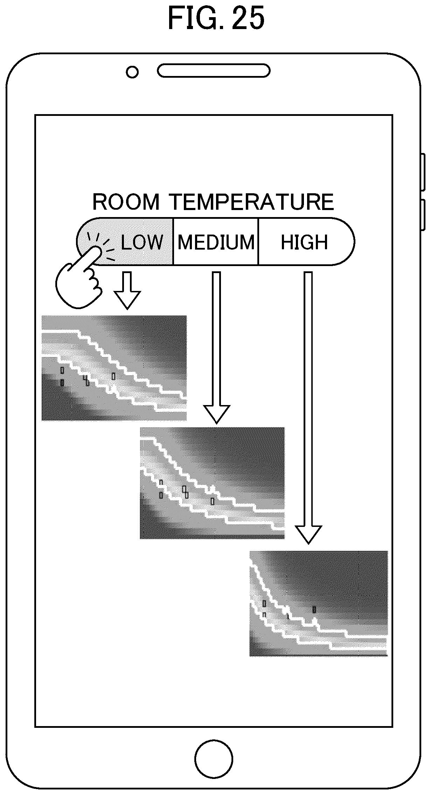

Next, a bathing information system according to a fourth embodiment of the present invention will be described with reference to FIG. 25.

The bathing information system of the present embodiment is different from the third embodiment described above in that temperature in a room (bath room) in which a bathtub is provided is acquired as objective information. Hereinafter, only differences of the fourth embodiment of the present invention from the third embodiment will be described, and description of a similar configuration, operation, and effect will be omitted.

FIG. 25 is a view schematically illustrating an operation of the bathing information system according to the fourth embodiment of the present invention.

In the bathing information system of the present embodiment, the temperature of warm water in the bathtub, the bathing time, and the temperature in the bath room are acquired as the objective information each time a specified bather bathes. After each bathing, the bather inputs "feeling of warmth" in the bathing in five levels of "too hot", "slightly hot", "comfortable", "slightly cold" and "too cold". Program installed on a smartphone ties and stores the acquired objective information and the subjective information input by the bather. The program further generates such a heat map as shown in FIG. 19 for each temperature in the bath room. In the present embodiment, it is presumed that when the temperature in the bath room is less than 24°C, the room temperature is "low", when the temperature is 24°C or more and less than 28°C, the room temperature is "medium", and when the temperature is 28°C or more, the room temperature is "high". Then, the program presumes three types of heat maps corresponding to the room temperatures "low", "medium" and "high", respectively.

When the bather bathes and, as shown in FIG. 25, taps any of "low", "medium", and "high" according to the temperature in the bath room on that day, the heat map presumed corresponding to the room temperature is displayed on the screen of the smartphone. The operation after displaying the heat map is the same as in the third embodiment described above, and hence description is omitted. Alternatively, the present invention may be configured to automatically display a heat map corresponding to room temperature based on the temperature detected by the temperature sensor built in the smartphone. Furthermore, in the present embodiment, a heat map (not shown) displayed on the screen of the remote controller 9 is also selected according to the temperature in the bath room, and the same information as in the third embodiment is displayed.

According to the bathing information system of the fourth embodiment of the present invention, examples of the value of the objective information include the temperature in the room in which the bathtub is provided, and it is therefore possible to precisely recommend the bathing conditions that can satisfy the bather, even if subjectivity (satisfaction level) of the bather changes depending on season, weather on the day of bathing, or the like.

Furthermore, in the fourth embodiment described above, the temperature in the bath room is added as the objective information, and as a modification, the present invention may be configured to acquire a water level in the bathtub as objective information in place of the temperature in the bath room. In this case, a water level gauge (not shown) for detecting the water level in the bathtub is provided, and a detection signal of the water level gauge is acquired as objective information. That is, even when the temperature of warm water in the bathtub and the bathing time are the same, the bather warms well in an elevated water level of warm water in the bathtub, and the bather warms less in a low water level of warm water. In this modification, the program generates such a heat map as shown in FIG. 19 for each water level in the bathtub. The present embodiment classifies the water level in the bathtub into three levels of "low", "medium", and "high" and presumes three corresponding types of heat maps.

When the bather bathes and, as shown in FIG. 25, taps any of "low", "medium", and "high" according to the water level in the bathtub on that day, the heat map presumed corresponding to the water level is displayed on the screen of the smartphone. The operation after displaying the heat map is the same as in the third embodiment described above, and hence description is omitted. Alternatively, the present invention may be configured to transmit the water level detected by the water level gauge (not shown) to the smartphone and automatically display a heat map corresponding to the water level. Furthermore, in the present modification, a heat map (not shown) displayed on the screen of the remote controller 9 is also selected according to the water level in the bathtub, and the same information as in the third embodiment is displayed.

According to the present modification including the configuration described above, since the examples of the value of the objective information include the water level of warm water in the bathtub, a difference in feeling of warmth felt by the bather between half-body bathing and full-body bathing can be added to presumption of subjective information, and the bathing conditions that can satisfy the bather can be precisely recommended.

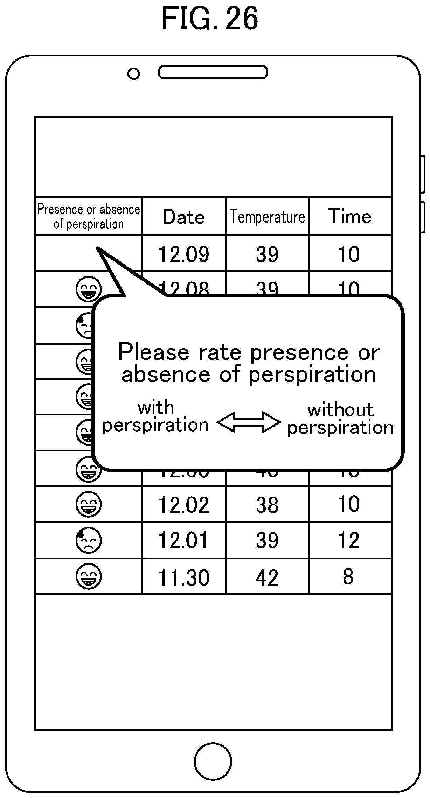

Next, a bathing information system according to a fifth embodiment of the present invention will be described with reference to FIGS. 26 to 28.

The bathing information system of the present embodiment is different from the third embodiment described above in that a bather inputs, as subjective information, whether the bather perspires after exiting a bathtub. Hereinafter, only differences of the fifth embodiment of the present invention from the third embodiment will be described, and description of a similar configuration, operation, and effect will be omitted.

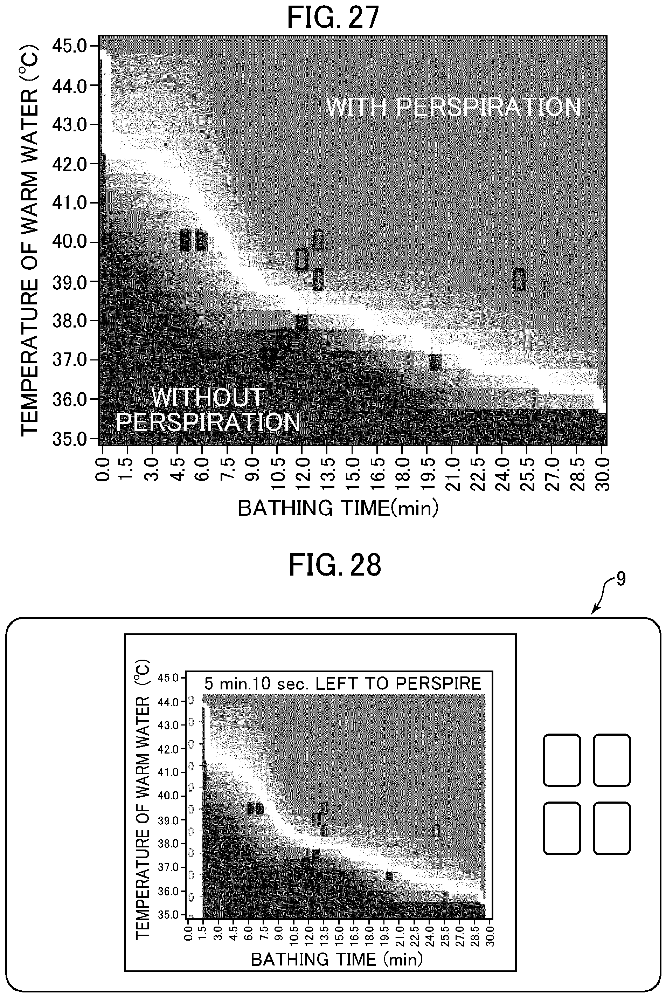

FIG. 26 is a view illustrating an example of a screen in which the bather inputs the subjective information, in the bathing information system according to the fifth embodiment of the present invention. FIG. 27 is a diagram illustrating an example of a screen displayed on bather's smartphone before bathing, in the bathing information system according to the fifth embodiment of the present invention. FIG. 28 is a view illustrating an example of a screen displayed on a remote controller 9 during bathing, in the bathing information system according to the fifth embodiment of the present invention.

As shown in FIG. 26, in the bathing information system of the present embodiment, a temperature of warm water in a bathtub and a bathing time are acquired as objective information each bathing time of a specified bather. After each bathing, the bather inputs presence or absence of perspiration of the bather after the bathing, that is, "with perspiration" or "without perspiration" as subjective information. Program installed on the smartphone ties and stores the acquired objective information and the subjective information input by the bather. The program further presumes a heat map shown in FIG. 27. As shown in FIG. 27, this heat map presumes whether the bather perspires after exiting the bathtub with respect to the temperature of warm water and the bathing time that are the objective information.

In the bathing information system of the present embodiment, when the bather activates predetermined application program on the smartphone before the bathing, the heat map presumed based on the stored bathing history as shown in FIG. 27 is displayed. Here, when a set temperature of a bathtub apparatus to enter is set to, for example, 39°C and warm water at 39°C is stored in the bathtub, the bather taps around 39°C on the vertical axis of the heat map. Consequently, when the temperature of warm water is 39°C, a minimum bathing time for which the subjective information is presumed to be "with perspiration" is calculated by the program activated on the smartphone based on the heat map. The calculated bathing time is, for example, displayed below the heat map on the screen of the smartphone as "bathing for about nine minutes or more achieves perspiration" (not shown).

In contrast, if the bather wishes to "bathe for about 15 minutes today" before bathing, the bather taps around 15 minutes on the horizontal axis of the heat map. Consequently, when the bathing time is 15 minutes, the minimum temperature of warm water that is presumed to have subjective information "with perspiration" is calculated based on the heat map. The calculated temperature of warm water is, for example, displayed below the heat map on the screen of the smartphone as "setting the set temperature to about 38.5°C or more achieves perspiration" (not shown). If the bather wishes to perspire, the bather can perspire when exiting the bathtub after bathing for a desired bathing time, by changing the set temperature of the bathtub apparatus based on this display.

In contrast, when the bather is in the bathtub, a screen shown in FIG. 28 is displayed on the remote controller 9. As shown in FIG. 28, the display section of the remote controller 9 displays how many more minutes to bathe before perspiring after exiting the bathtub. The example shown in FIG. 28 displays that bathing further for five minutes and ten seconds or more achieves perspiration after exiting the bathtub. Specifically, based on the temperature of warm water stored in the bathtub and elapsed time after the bather enters the bathtub, the program calculates how many more minutes to bathe is required before perspiring after exiting from the bathtub, and displays a remaining time on the screen of the remote controller 9. Consequently, if the bather wishes to perspire, the bather continues bathing until the remaining time on the screen of the remote controller 9 reaches at least 0. If the bather does not wish to perspire, the bather should exit the bathtub before the remaining time on the screen of the remote controller 9 reaches 0.

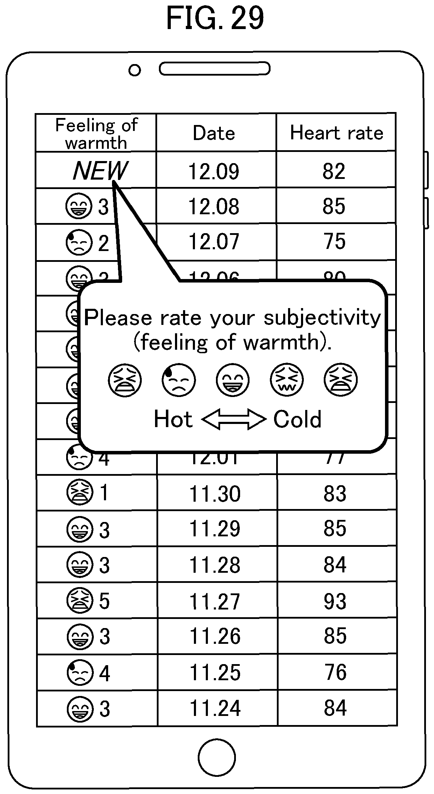

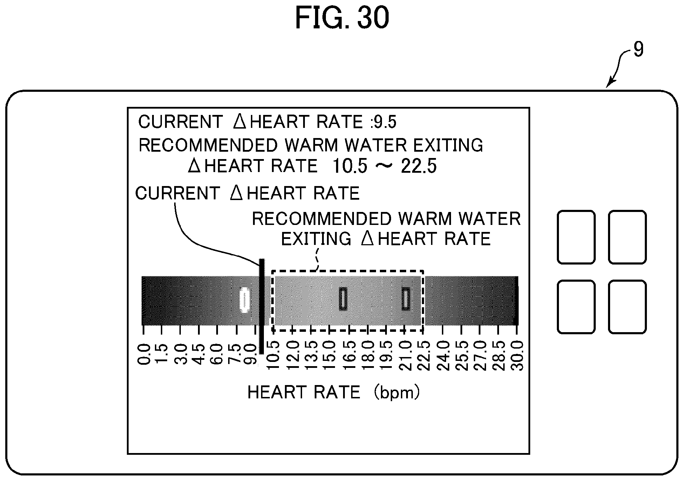

Next, a bathing information system according to a sixth embodiment of the present invention will be described with reference to FIGS. 29 and 30.

The bathing information system of the present embodiment is different from the third embodiment described above in that a heart rate of a bather is acquired as objective information, and a heart rate gauge is displayed on a remote controller. Hereinafter, only differences of the sixth embodiment of the present invention from the third embodiment will be described, and description of a similar configuration, operation, and effect will be omitted.

FIG. 29 is a view illustrating an example of a screen in which the bather inputs subjective information, in the bathing information system according to the sixth embodiment of the present invention. FIG. 30 is a view illustrating an example of a gauge displayed on a remote controller screen in a bath room.

In the bathing information system of the present embodiment, each time a specified bather enters a bathtub, heart rates of the bather at start of bathing and after exiting the bathtub are acquired as objective information (only the heart rate after the exiting the bathtub is displayed in the display screen of FIG. 29). After each bathing, the bather inputs "feeling of warmth" in the bathing in five levels of "too hot", "slightly hot", "comfortable", "slightly cold", and "too cold". Program installed on a smartphone ties and stores the acquired objective information and the subjective information input by the bather. The program further presumes how many times a heart rate per minute (Δheart rate) rises from the start of bathing to the exiting the bathtub for obtaining the "comfortable" "feeling of warmth", based on the acquired objective information and the input subjective information.

Then, as shown in FIG. 30, during the bathing of the bather, the remote controller 9 displays "current Δheart rate" (how many times the heart rate has risen after the start of bathing) and "recommended warm water exiting Δheart rate" (how many times the heart rate per minute rises for obtaining the "comfortable" "feeling of warmth" when the bather exits the bathtub). Furthermore, below this display, an indication line indicating the current Δheart rate, and a range of recommended warm water exiting Δheart rate are displayed on the heart rate gauge. The example shown in FIG. 30 shows, by numerical values and the gauge, that the heart rate currently rises 9.5 times from the start of bathing and that the bather can obtain the "comfortable" "feeling of warmth" by exiting the bathtub when the heart rate rises 10.5 to 22.5 times from the start of bathing. The bather can obtain the "comfortable" "feeling of warmth" by exiting the bathtub when the indication line of the "current heart rate" displayed on the heart rate gauge falls within a frame of "recommended heart rate".

According to the bathing information system of the sixth embodiment of the present invention, it is possible to obtain the "comfortable" "feeling of warmth" based on the objective information of the heart rate.

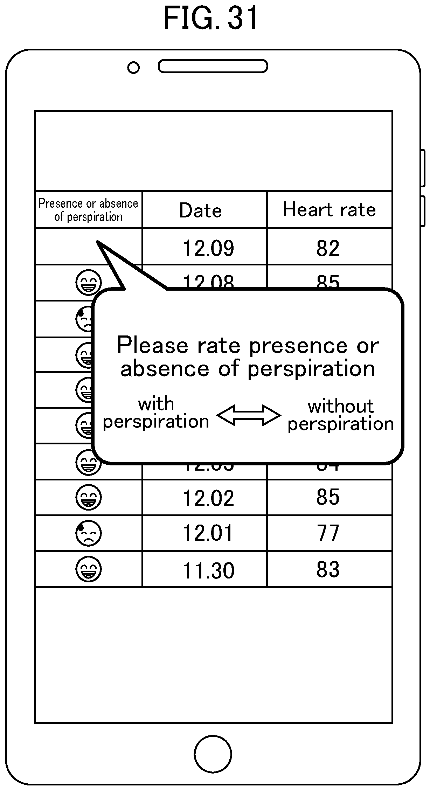

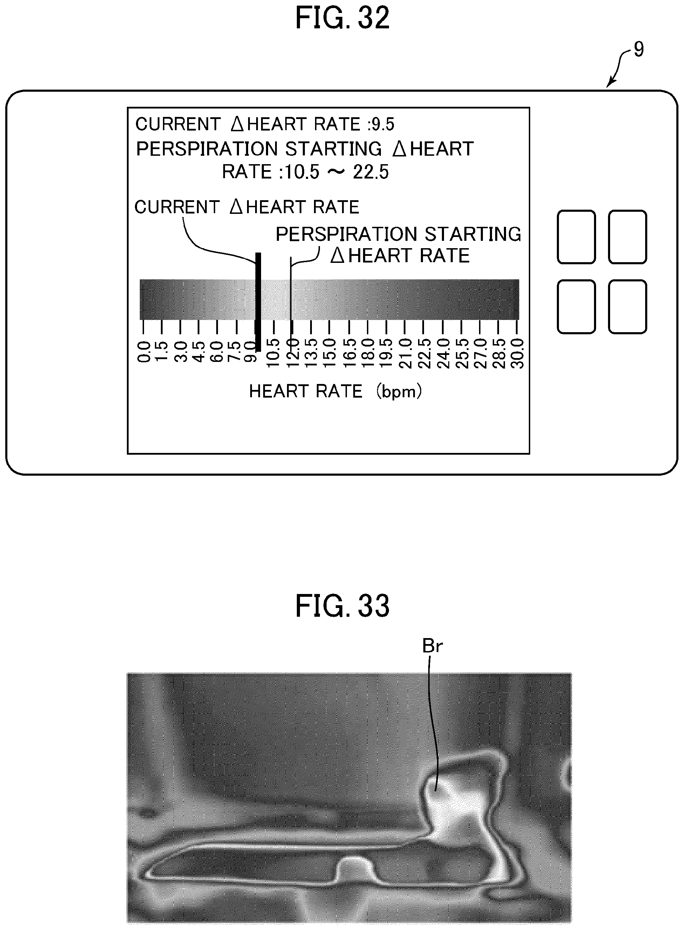

Next, a bathing information system according to a seventh embodiment of the present invention will be described with reference to FIGS. 31 and 32.

The bathing information system of the present embodiment is different from the sixth embodiment described above in that presence or absence of perspiration of a bather is input as subjective information. Hereinafter, only differences of the seventh embodiment of the present invention from the sixth embodiment will be described, and description of a similar configuration, operation, and effect will be omitted.

FIG. 31 is a view illustrating an example of a screen in which a bather inputs subj ective information, in the bathing information system according to the seventh embodiment of the present invention. FIG. 32 is a view illustrating an example of a gauge displayed on a remote controller screen in a bath room.

In the bathing information system of the present embodiment, each time a specified bather enters a bathtub, heart rates when the bather enters and exits the bathtub are acquired as objective information (heart rate only when the bather exits the bathtub is displayed on the display screen of FIG. 31). The bather inputs whether the bather perspires after each bathing. Program installed on a smartphone ties and stores acquired objective information and subjective information input by the bather. The program further presumes how many times a heart rate per minute (Δheart rate) rises from when the bather enters the bathtub to when the bather exits the bathtub to perspire after exiting the bathtub, based on the acquired objective information and the input subjective information.

Next, as shown in FIG. 32, while the bather is bathing, the remote controller 9 displays "current Δheart rate" (how many times the heart rate rises after the start of bathing) and "perspiration starting Δheart rate" (how many times the heart rate per minute rises for the bather to perspire after exiting the bathtub). Furthermore, below this display, indication lines indicating the current Δheart rate and a minimum Δheart rate for the bather to perspire after exiting the bathtub are displayed on a heart rate gauge. The example shown in FIG. 32 shows, by numerical values and the gauge, that the heart rate currently rises 9.5 times from the start of bathing and that when the heart rate rises 12.0 or more times from the start of bathing, the bather perspires after exiting the bathtub. If the bather wishes to perspire after exiting the bathtub, the bather exits the bathtub when the indication line of the "current heart rate" displayed on the heart rate gauge exceeds the indication line of "perspiration starting heart rate". In addition, if the bather does not wish to perspire after exiting the bathtub, the bather exits the bathtub before the indication line of the "current heart rate" displayed on the heart rate gauge exceeds the indication line of the "perspiration starting heart rate".

According to the bathing information system of the seventh embodiment of the present invention, it is possible to recognize whether the bather perspires after exiting the bathtub, based on the objective information of the heart rate.

Next, a bathing information system according to an eighth embodiment of the present invention will be described with reference to FIGS. 33 to 35.

The bathing information system of the present embodiment is different from the third embodiment described above in that a body temperature of a bather is acquired as objective information, and a body temperature gauge is displayed on a remote controller. Hereinafter, only differences of the eighth embodiment of the present invention from the third embodiment will be described, and description of a similar configuration, operation, and effect will be omitted.

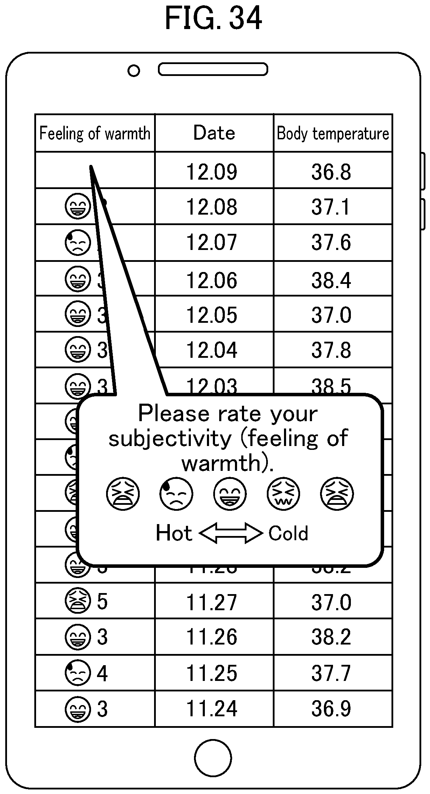

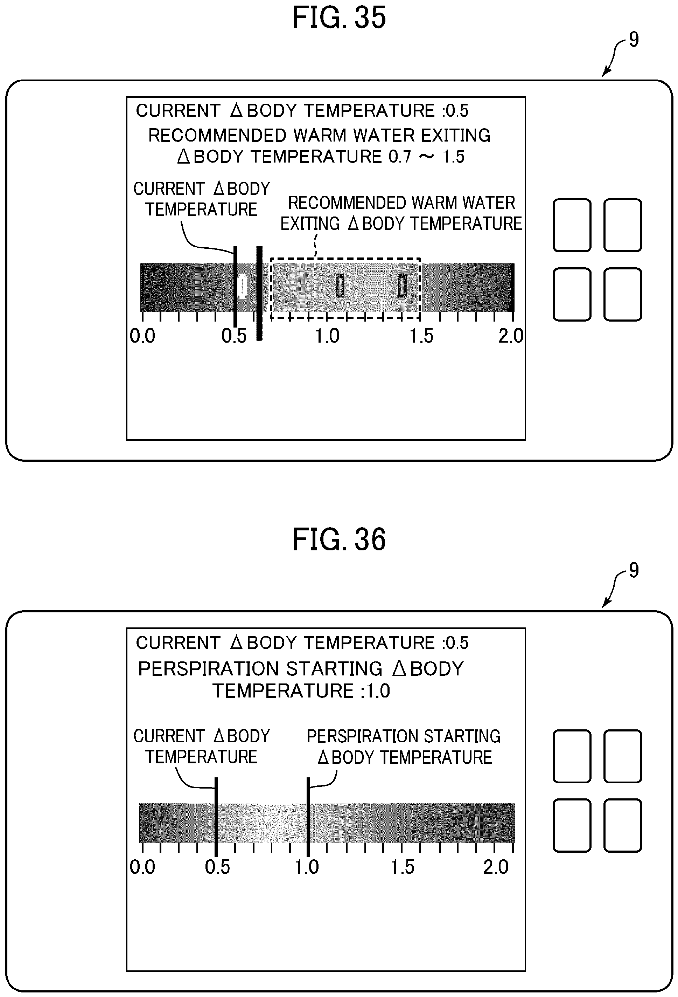

FIG. 33 is a diagram illustrating an example of an image captured by a thermo-sensor for acquiring the body temperature of the bather, in the bathing information system according to the eighth embodiment of the present invention. FIG. 34 is a view illustrating an example of a screen in which the bather inputs subjective information. FIG. 35 is a view illustrating an example of a gauge displayed on a remote controller screen in a bath room.

In the bathing information system of the present embodiment, as shown in FIG. 33, an image of a bather bathing in the bathtub is captured by the thermo-sensor (not shown). By analyzing this captured image, a temperature of a portion of a brow Br of the bather is acquired as objective information on the body temperature of the bather. Therefore, as shown in FIG. 34, each time a specified bather enters the bathtub, body temperatures of the bather when the bather enters and exits the bathtub are acquired (only the body temperature when the bather exits the bathtub is displayed on the display screen of FIG. 34) as obj ective information. After each bathing, the bather inputs "feeling of warmth" in the bathing in five levels of "too hot", "slightly hot", "comfortable", "slightly cold"," and "too cold". Program installed on a smartphone ties and stores the acquired objective information and the subjective information input by the bather. The program further presumes how many degrees the body temperature (Δbody temperature) rises from when the bather enters the bathtub to when the bather exits the bathtub to obtain "comfortable" "feeling of warmth", based on the acquired objective information and the input subjective information.

Then, as shown in FIG. 35, during the bathing of the bather, a remote controller 9 displays "current Δbody temperature" (how many degrees the body temperature rises after the start of bathing) and "recommended warm water exiting Δbody temperature" (how many degrees the body temperature rises so that the bather can obtain the "comfortable" "feeling of warmth" on exiting the bathtub). Furthermore, below this display, the body temperature gauge displays an indication line indicating the current Δbody temperature, and a range of the recommended warm water exiting Δbody temperature. The example shown in FIG. 35 shows, by numerical values and the gauge, that the body temperature currently rises by 0.5°C from the start of bathing and that the bather can obtain the "comfortable" "feeling of warmth" on exiting the bathtub when the body temperature rises by 0.7°C to 1.5°C from the start of bathing. The bather can obtain the "comfortable" "feeling of warmth" by exiting the bathtub when the indication line of the "current Δbody temperature" displayed on the body temperature gauge falls within a frame of the "recommended warm water exiting Δbody temperature".

According to the bathing information system of the eighth embodiment of the present invention, the bather can obtain the "comfortable" "feeling of warmth" based on the objective information of body temperature.

Next, a bathing information system according to a ninth embodiment of the present invention will be described with reference to FIG. 36.

The bathing information system of the present embodiment is different from the eighth embodiment described above in that presence or absence of perspiration of a bather is input as subjective information. Hereinafter, only differences of the ninth embodiment of the present invention from the eighth embodiment will be described, and description of a similar configuration, operation, and effect will be omitted.

FIG. 36 is a view illustrating an example of a gauge displayed on a remote controller screen in a bath room, in the bathing information system according to the ninth embodiment of the present invention.

In the bathing information system of the present embodiment, an image of the bather bathing in a bathtub is captured by a thermo-sensor (not shown). By analyzing this captured image, a temperature of a portion of a brow Br of the bather is acquired as objective information on a body temperature of the bather. Consequently, each time a specified bather enters the bathtub, body temperatures when the bather enters and exits the bathtub are acquired as the objective information. After each bathing, the bather inputs whether the bather has perspired after exiting the bathtub. Program installed on a smartphone ties and stores the acquired obj ective information and the subj ective information input by the bather. The program further presumes by how many degrees the body temperature (Δbody temperature) rises from when the bather enters the bathtub to when the bather exits the bathtub to perspire, based on the acquired objective information and the input subjective information.

Then, as shown in FIG. 36, during the bathing of the bather, a remote controller 9 displays "current Δbody temperature" (how many degrees the body temperature rises after the start of bathing) and "perspiration starting Δbody temperature" (how many degrees the body temperature rises so that the bather perspires on exiting the bathtub). Furthermore, below this display, a body temperature gauge displays thereon an indication line indicating the current Δbody temperature, and an indication line indicating Δbody temperature to start perspiration. The example shown in FIG. 36 shows, by numerical values and the gauge, that the body temperature currently rises by 0.5°C from the start of bathing and that the bather perspires after exiting the bathtub when the body temperature rises by 0.7°C or more from the start of bathing. If the bather wishes to perspire after exiting the bathtub, the bather exits the bathtub after the indication line of the "current body temperature" displayed on the body temperature gauge exceeds an indication line of "perspiration starting body temperature". If the bather does not wish to perspire after exiting the bathtub, the bather exits the bathtub before the indication line of the "current body temperature" displayed on the body temperature gauge exceeds the indication line of the "perspiration starting body temperature".

According to the bathing information system of the ninth embodiment of the present invention, it is possible to recognize whether the bather perspires after exiting the bathtub, based on the objective information of the heart rate.

Although the embodiments of the present invention have been described above, various changes can be made to the above-described embodiments. In particular, the present invention may be configured by appropriately combining the configurations provided in the above-described respective embodiments.

### Reference Signs List

1: bathing information system
2: bathtub apparatus
4: bathtub body
42: bather specifying unit
44: information acquisition unit
50: program
101: bathing information system
102: bathtub apparatus
150: program
A: objective information
B: subjective information
C: history information
D: recommended information
E: recommended range information
R: bath room

## Claims

1. A bathing information system for acquiring information on a bather bathing in a bathtub, comprising:
a bather specifying unit that specifies who the bather is for specific bathing,
an information acquisition unit that acquires objective information on the bathing of the bather, and
program that controls a controller to store subjective information input by the bather in the controller,
the program causing the controller to store a bathing history obtained by associating the objective information acquired by the information acquisition unit regarding the specific bathing of the specified bather with the subjective information input by the bather specified for the specific bathing, and
causing the controller to implement an output function of outputting, to an output device, history information based on a plurality of bathing histories for the specified bather.

2. The bathing information system according to claim 1, wherein the objective information includes a temperature of warm water in the bathtub and a bathing time.

3. The bathing information system according to claim 1, wherein the objective information includes biological information of the bather.

4. The bathing information system according to claim 1, wherein the program further causes the controller to implement a function of outputting, to the output device, preset recommended information together with the history information.

5. The bathing information system according to claim 1, wherein the history information includes recommended range information determined based on the plurality of bathing histories.

6. The bathing information system according to any one of claims 1 to 5, wherein the output function includes a first output function and a second output function, and
the program causes the controller to store a first bathing history obtained by associating first objective information selected for a first analysis application from the objective information with first subjective information input for the first analysis application from the subjective information and causes the controller to implement the first output function of outputting, to the output device, first history information based on a plurality of first bathing histories for a specified bather,
the program causes the controller to store a second bathing history obtained by associating second objective information selected for a second analysis application from the objective information with second subjective information input for the second analysis application from the subjective information and causes the controller to implement the second output function of outputting, to the output device, second history information based on a plurality of second bathing histories for the specified bather, and
the program further causes the controller to implement a function of switching between the first output function and the second output function.

7. A bathing information system for acquiring information on a bather bathing in a bathtub, comprising:
a bather specifying unit that specifies who the bather is for specific bathing,
an information acquisition unit that acquires objective information on bathing of the bather,
an input unit that accepts input of subjective information,
a controller that stores the objective information and the subjective information; and
a display that displays information received from the controller, wherein the controller stores a bathing history obtained by associating the objective information acquired for the specific bathing of the specified bather by the information acquisition unit with the subjective information input in the input unit regarding the bather specified for the specific bathing, and
displays, on the display, history information based on a plurality of bathing histories for the specified bather.

8. The bathing information system according to claim 1, wherein the program presumes subjective information corresponding to a value of objective information to which any subjective information is not tied, based on objective information on at least one bathing, and subjective information tied to the value of the objective information and causes the output device to output presumed subjective information.

9. The bathing information system according to claim 8, wherein examples of the value of the objective information include a temperature of warm water in the bathtub, and a bathing time.

10. The bathing information system according to claim 9, wherein the examples of the value of the objective information further include a temperature in a room in which the bathtub is provided.

11. The bathing information system according to claim 9, wherein the examples of the value of the objective information further include a water level of warm water in the bathtub.
